# EUROPÄISCHE PATENTANMELDUNG

(11) **EP 2 105 126 A1**
(43) Veröffentlichungstag der Anmeldung: **30.09.2009**
(21) Anmeldenummer: 08153273.1
(22) Anmeldetag: 26.03.2008
(51) Int. Cl.: A61K 8/87, A61Q 1/02, A61Q 1/06, A61Q 1/10, A61Q 19/00, A61Q 19/04

(54) **Dekorative kosmetische Zusammensetzungen**

(71) Anmelder: Bayer MaterialScience AG, 51368 Leverkusen (DE)
(72) Erfinder: Die Erfindernennung liegt noch nicht vor

(57) **Zusammenfassung**

Die vorliegende Erfindung betrifft dekorative kosmetische Zusammensetzungen, enthaltend spezielle Polyurethane bzw. wässrige Dispersionen davon und dekorative Effekte bereitstellende Bestandteile.

## Beschreibung

Die vorliegende Erfindung betrifft dekorative kosmetische Zusammensetzungen, enthaltend spezielle Polyurethane bzw. wässrige Dispersionen davon und dekorative Effekte bereitstellende Bestandteile.

Eine dekorative kosmetische Zusammensetzung wie die der Erfindung dient der dekorativen, insbesondere farblichen Gestaltung der menschlichen Haut, Schleimhaut, Semi-Schleimhaut und des Haars, insbesondere der Augenlider und der Augenbrauen. Der dekorative Effekt wird durch mindestens einen effektgebenden Bestandteil erzielt. Die erfindungsgemäße dekorative Zusammensetzung kann beispielsweise ein Gesichts-Make-up (Foundation), eine getönte (Tages)Creme, ein Blush, ein Rouge, eine Wimperntusche, ein Eyeliner, ein Kajal, ein Lidschatten, ein Lippenstift, ein Lip Gloss sein. Diese speziellen kosmetischen Formulierungen dienen der farblichen Veränderung oder zum Schminken des Körpers, um beispielsweise Augenringe, einen inhomogenen Teint oder weitere Unvollkommenheiten der Haut wie Rötungen, Flecken, Falten oder Pickel zu überdecken und somit dem Anwender ein ästhetischeres Aussehen zu verleihen. Die vorstehend genannte Liste von dekorativen Produkten ist selbstverständlich nicht limitierend. Nagellackzusammensetzungen sind von dieser Anmeldung ausgeschlossen.

Die dekorativen kosmetischen Zusammensetzungen enthalten zweckmäßig einen oder mehrere Farbstoffe, die beispielsweise aus der Gruppe von löslichen Farbstoffen, anorganische Pigmenten wie zum Beispiel Eisenoxide, Chromoxide, Ultramarin, Manganviolett, organischen Pigmenten und Perlmutt ausgewählt werden. Abhängig von der Formulierungsform können solche dekorativen kosmetischen Zusammensetzungen aus bis zu 80 Gew.-% Farbstoffen und Füllstoffen, bezogen auf das Gesamtgewicht der Zusammensetzung bestehen.

Die Verbraucher wünschen sich bei Anwendung dekorativer kosmetischer Formulierungen naturgemäß einen langanhaltenden dekorativen Effekt. Insbesondere erwarten die Verbraucher eine gute Beständigkeit gegenüber Wasser, wie während des Badens oder beim Duschen, Tränen oder Schweiß wie insbesondere während sportlicher Aktivitäten.

Zur Verbesserung der Beständigkeit von dekorativen Produkten gegenüber Wasser, Tränen oder Schweiß (oft Wasserfestigkeit genannt) werden filmbildende Polymere eingesetzt. Als filmbildende Polymere werden bevorzugt Polymere auf Basis von Acrylaten oder Vinylpyrrolidone gewählt. Die Nachteile solcher filmbildenden Polymere sind dem Fachmann bekannt. Die Acrylatpolymere bilden nämlich harte und spröde Filme. Daraus resultiert ein unangenehmes Gefühl beim Tragen des Produktes. Wegen des klebrigen Hautgefühles, können die Vinylpyrrolidone nur in begrenzten Konzentrationen eingesetzt werden.

Auch die Anwendung von Polyurethandispersionen ist in der dekorativen Kosmetik bekannt. So beschreibt die US 2007/0154440 die Anwendung eines filmbildenden Polyurethans mit einem Molekulargewicht von mindestens 50000 in einer kosmetischen Formulierung zur Erstellung eines langanhaltenden Films auf der Haut. Die FR 2832058 beschreibt die Verwendung einer wässrigen Polyurethandispersion in einer Eyeliner-Zusammensetzung. Die US 20070025943 beschreibt die Kombination von einem filmbildenden (Meth)acrylate Coplymer und einem filmbildenden Polyurethane in einer kosmetischen Zusammensetzung. Die EP 0775483 (DE 69621104) beschreibt den Einsatz einer wässrigen Dispersion von synthetischen, filmbildenden Polymerteilchen in einer Zusammensetzung zum Schminken der Lippen. Die EP 1010418 beschreibt der Einsatz einer wässrigen Polyurethandispersion in einer wachs-freien Mascara-Zusammensetzung. Die WO 2003039445 beschreibt die Verwendung einer wässrigen Polyurethandispersion in einer kosmetischen Zusammensetzung. Die WO02070577A1 (US 2004/0197293) beschreibt anionische Polyurethane, welche in kosmetischen Zusammensetzungen verwendet werden können. Konkrete Beispiele kosmetischer Zusammensetzungen werden jedoch nicht beschrieben. Die beschriebenen anionischen Polyurethane weisen eine vergleichsweise geringe Wasserfestigkeit auf und bilden wässrige Polyurethandispersionen vergleichsweise hoher Viskosität, was ihre Verarbeitung erschwert.

Tragekomfort, insbesondere verringerte Klebrigkeit, Beständigkeit, insbesondere Wasserfestigkeit und Glanz der polyurethanhaltigen kosmetischen Zusammensetzungen, insbesondere für die Bereitstellung dekorativer Effekte, aus dem Stand der Technik geben somit noch Raum für Verbesserungen. Des weiteren weisen die im Stand der Technik verwendeten wässrigen Polyurethandispersionen vielfach eine nachteilig hohe Viskosität auf, was ihre Verarbeitbarkeit bzw. ihre Einarbeitbarkeit in kosmetische Formulierungen erschweren kann.

Der vorliegenden Erfindung liegt daher die Aufgabe zugrunde, eine dekorative kosmetische Zusammensetzung bereitzustellen, welche einen hohen Tragekomfort, insbesondere eine verringerte Klebrigkeit, eine hohe Beständigkeit, insbesondere Wasserfestigkeit und verbesserte Glanzeigenschaften aufweist. Des weiteren sollten die erfindungsgemäß verwendeten wässrigen Polyurethandispersionen eine vergleichsweise geringe Viskosität aufweisen, so dass sie sich leicht in kosmetische Zusammensetzungen für dekorative Zwecke einarbeiten lassen.

Überraschend wird die Aufgabe gelöst durch den Einsatz von speziellen Polyurethanen bzw. wässrigen Dispersionen davon, erhältlich durch Umsetzung eines oder mehrerer wasserunlöslicher, nicht wasserdispergierbarer, isocyanatfunktioneller Po-Iyurethanprepolymere A) mit einer oder mehreren aminofunktionellen Verbindungen B).

Die vorliegende Erfindung stellt somit eine dekorative kosmetische Zusammensetzung bereit, enthaltend mindestens ein Polyurethan, erhältlich durch Umsetzung eines oder mehrerer wasserunlöslicher, nicht wasserdispergierbarer, isocyanatfunktioneller Polyurethanprepolymere A) mit einer oder mehreren aminofunktionellen Verbindungen B).

Weiterhin stellt die vorliegende Erfindung eine dekorative kosmetische Zusammensetzung bereit, enthaltend mindestens ein Polyurethan, erhältlich durch Umsetzung eines oder mehrerer isocyanatfunktioneller Polyurethanprepolymere A), welche im Wesentlichen weder ionische noch ionogene Gruppen aufweisen, mit einer oder mehreren aminofunktionellen Verbindungen B).

Im Rahmen der Erfindung bedeutet der Begriff "wasserunlösliches, nicht wasserdispergierbares Polyurethanprepolymer" insbesondere, dass die Wasserlöslichkeit des erfindungsgemäß verwendeten Prepolymers bei 23°C weniger als 10 g / Liter, bevorzugter weniger als 5 g / Liter beträgt und das Prepolymer bei 23° keine sedimentationsstabile Dispersion in Wasser, insbesondere entionisiertem Wasser, ergibt.

Mit anderen Worten setzt sich das Prepolymer, beim Versuch es in Wasser zu dispergieren, ab.

Bevorzugt weist das erfindungsgemäß verwendete Polyurethanprepolymere A) endständige Isocyanatgruppen auf, d.h. die Isocyanatgruppen liegen an den Kettenenden des Prepolymers. Besonders bevorzugt weisen sämtliche Kettenenden eines Polymers Isocyanatgruppen auf.

Weiterhin weist das erfindungsgemäß verwendete Polyurethanprepolymere A) bevorzugt im Wesentlichen weder ionische noch ionogene (zur Bildung von ionischen Gruppen befähigte) Gruppen auf, d.h. der Gehalt an ionischen und ionogenen Gruppen liegt zweckmäßig unter 15 Milliequivalenten pro 100 g Polyurethanprepolymer A), bevorzugt unter 5 Milliequivalenten, besonders bevorzugt unter einem Milliequivalente und ganz besonders bevorzugt unter 0,1 Milliequivalenten pro 100 g Polyurethanprepolymer A).

Die aminofunktionellen Verbindungen B) werden bevorzugt aus primären und/oder sekundären Aminen und/oder Diaminen ausgewählt. Insbesondere umfassen die aminofunktionellen Verbindungen B) mindestens ein Diamin. Die aminofunktionellen Verbindungen B) werden bevorzugt aus aminofunktionellen Verbindungen B2), die ionische oder ionogene Gruppe aufweisen, und aminofunktionellen Verbindungen B1), die keine ionische oder ionogene Gruppe aufweisen, ausgewählt.

In einer besonders bevorzugten Ausführungsform der Erfindung umfassen die aminofunktionellen Verbindungen B) wenigstens eine aminofunktionelle Verbindung B2), die ionische und/oder ionogene (ionenbildende) Gruppen aufweist. Besonders bevorzugt wird als ionische und/oder ionogene Gruppe die Sulfonat- bzw. die Sulfonsäuregruppe, noch bevorzugter die Natriumsulfonatgruppe verwendet.

In einer weiteren bevorzugten Ausführungsform der Erfindung umfassen die aminofunktionellen Verbindungen B) sowohl aminofunktionelle Verbindungen B2), die ionische und/oder ionogene Gruppe aufweisen, als auch aminofunktionellen Verbindungen B1), die keine ionische oder ionogene Gruppe aufweisen.

Polyurethane im Sinne der Erfindung sind demnach polymere Verbindungen, die mindestens zwei bevorzugt mindestens drei Urethan-Gruppen-haltige Wiederholungseinheiten aufweisen: 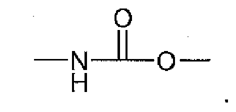 Erfindungsgemäß sind auch solche Polyurethane eingeschlossen, die herstellungsbedingt auch Harnstoff-Gruppen-ha!t!ge Wiederholungseinheiten: 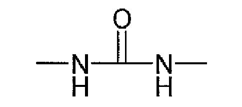 aufweisen, wie sie insbesondere bei der Umsetzung der isocyanatterminierten Prepolymere A) mit den aminofunktionellen Verbindungen B) gebildet werden.

Bei den erfindungsgemäßen dekorativen kosmetischen Zusammensetzungen kann es sich um wasser-enthaltende, d.h. wässrige Zusammensetzungen handeln, in denen das Polyurethan dispergiert, d.h. im Wesentlichen nicht gelöst vorliegt. Wasser kann neben wahlweise vorhandenen anderen flüssigen Medien, wie beispielsweise Lösungsmittel, den Hauptbestandteil (> 50 Gew.-%) der Dispergiermedien, bezogen auf die Gesamtmenge der flüssigen Dispergiermedien in den erfindungsgemäßen kosmetischen Zusammensetzungen, gegebenenfalls auch das alleinige flüssige Dispergiermedium sein.

Die erfindungsgemäßen dekorativen kosmetischen Zusammensetzungen weisen bevorzugt einen Gehalt an flüchtigen organischen Verbindungen (VOC) von weniger als 80 Gew.-%, bevorzugter von weniger als 55 Gew.-%, noch bevorzugter von weniger als 40 Gew.-% bezogen auf die dekorative kosmetische Zusammensetzung auf.

Die zur Herstellung der erfindungsgemäßen dekorativen kosmetischen Zusammensetzungen verwendeten wässrigen Polyurethandispersionen weisen bevorzugt einen Gehalt an flüchtigen organischen Verbindungen (VOC) von weniger als 10 Gew.-%, bevorzugter von weniger als 3 Gew.-%, noch bevorzugter von weniger als 1 Gew.-% bezogen auf die auf wässrige Polyurethandispersion auf.

Die Bestimmung des Gehalts an flüchtigen organischen Verbindungen (VOC) erfolgt im Rahmen der vorliegenden Erfindung insbesondere durch gaschromatographische Analyse.

Die erfindungsgemäß verwendeten nicht wasserlöslichen und nichtwasserdispergierbaren, isocyanatfunktionellen Polyurethanprepolymere, weisen im Wesentlichen weder ionische noch ionogene Gruppen auf. Die Wasserunlöslichkeit bzw. fehlende Dispergierbakeit in Wasser bezieht sich auf entionisiertes Wasser ohne Zusatz von Tensiden. Im Rahmen der vorliegenden Erfindung bedeutet dies, dass der Anteil der ionischen und/oder ionogenen (ionen-bildenden) Gruppen, wie insbesondere anionischer Gruppen, wie Carboxylat oder Sulfonat, oder kationischer Gruppen weniger beträgt als 15 Milliequivalente pro 100 g Polyurethanprepolymer A), bevorzugt weniger als 5 Milliequivalente, besonders bevorzugt weniger als ein Milliequivalent und ganz besonders bevorzugt weniger als 0,1 Milliequivalente pro 100 g Polyurethanprepolymer A).

Im Falle saurer ionischer und/oder ionogener Gruppen beträgt die Säurezahl des Prepolymers zweckmäßig unter 30 mg KOH/g Prepolymer, bevorzugt unter 10 mg KOH/g Prepolymer. Die Säurezahl gibt die Masse Kaliumhydroxid in mg an, die zur Neutralisation von 1 g der zu untersuchenden Probe erforderlich ist (Messung nach DIN EN ISO 211). Die neutralisierten Säuren, also die entsprechenden Salze weisen naturgemäß keine oder eine reduzierte Säurezahl auf. Hier ist erfindungsgemäß die Säurezahl der korrespondierenden freien Säure entscheidend.

Die zur Herstellung der Polyurethane verwendeten Prepolymere A) sind bevorzugt erhältlich durch die Umsetzung von einem oder mehreren Polyolen, ausgewählt aus der Gruppe, die aus Polyether-Polyolen, Polycarbonatpolyolen, Polyether-Polycarbonat-Polyolen und/oder Polyesterpolyolen besteht, und Polyisocyanaten, wie weiter unten näher erläutert wird.

Die in den erfindungsgemäßen dekorativen kosmetischen Zusammensetzungen enthaltenen Polyurethane enthalten via das Prepolymer A) demnach bevorzugt mindestens eine Sequenz, die ausgewählt wird aus der Gruppe, die besteht aus: Polyether-, Polycarbonat-, Polyether-Polycarbonat- und Polyester-Sequenzen. Dies bedeutet erfindungsgemäß insbesondere, dass die Polyurethane Ethergruppen- und/oder Carbonatgruppen-haltige oder Estergruppen-Wiederholungseinheiten enthalten. Die Polyurethane können beispielsweise ausschließlich Polyether-Sequenzen oder ausschließlich Polycarbonatsequenzen oder ausschließlich Polyestersequenzen enthalten. Sie können aber auch sowohl Polyether- als auch Polycarbonat-Sequenzen aufweisen, wie sie beispielsweise bei der Herstellung von Polycarbonat-Polyolen unter Verwendung von Polyetherdiolen gebildet werden, wie unten noch ausführlich beschrieben wird. Zusätzlich können sie Polyether-Polycarbonat-Sequenzen aufweisen, welche aus der Verwendung von Polyether-Polycarbonat-Polyolen, wie weiter unten näher beschrieben, hervorgehen.

Besonders bevorzugte Polyurethane werden unter Verwendung von polymeren Polyether-Polyolen und/oder polymeren Polycarbonat-Polyolen und/oder Polyether-Polycarbonat-Polyolen oder Polyesterpolyolen erhalten, welche jeweils zahlenmittlere Molekulargewichte von bevorzugt etwa 400 bis etwa 6000 g/mol (hier und bei den folgenden Molekulargewichtangaben bestimmt durch Gelpermeationschromatographie gegenüber Polystyrol-Standard in Tetrahydofuran bei 23°C) aufweisen. Ihre Verwendung bei der Herstellung der Polyurethane bzw. Polyurethanprepolymere führt durch Umsetzung mit Polyisocyanaten zur Bildung entsprechender Polyether-und/oder Polycarbonat und/oder Polyether-Polycarbonat-Sequenzen oder Polyester-sequenzen in den Polyurethanen mit entsprechendem Molgewicht dieser Sequenzen. Besonders bevorzugt sind erfindungsgemäß Polyurethane, die aus polymeren Polyether-Diolen und/oder polymeren Polycarbonat-Diolen und/oder Polyether-Polycarbonat-Polyolen oder Polyesterpolyolen mit linearem Aufbau erhalten werden.

Die erfindungsgemäßen Polyurethane sind bevorzugt im Wesentlichen lineare Moleküle, können jedoch auch verzweigt sein, was weniger bevorzugt ist.

Das zahlenmittlere Molekulargewicht der erfindungsgemäß bevorzugt verwendeten Polyurethane beträgt beispielsweise etwa von 1000 bis 200000, bevorzugt von 5000 bis 150000.

Die in den erfindungsgemäßen dekorativen kosmetischen Zusammensetzungen enthaltenen Polyurethane werden den genannten Zusammensetzungen insbesondere als wässrige Dispersionen zugesetzt.

Bevorzugte erfindungsgemäß einzusetzenden Polyurethane bzw. Polyurethan-Dispersionen sind erhältlich, in dem
A) isocyanatfunktionelle Prepolymere aus
   A1) organischen Polyisocyanaten,
   A2) polymeren Polyolen, bevorzugt mit zahlenmittleren Molekulargewichten von 400 bis 8000 g/mol (hier und bei den folgenden Molekulargewichtangaben bestimmt durch Gelpermeationschromatographie gegenüber Polystyrol-Standard in Tetrahydofuran bei 23°C), bevorzugter 400 bis 6000 g/mol und besonders bevorzugt von 600 bis 3000 g/mol, und OH-Funktionalitäten von bevorzugt 1,5 bis 6, bevorzugter 1,8 bis 3, besonders bevorzugt von 1,9 bis 2,1,
   A3) gegebenenfalls hydroxyfunktionellen Verbindungen mit Molekulargewichten von bevorzugt 62 bis 399 g/mol, und
   A4) gegebenenfalls nichtionischen Hydrophilierungsmittein, hergestellt werden, und
B) deren freie NCO-Gruppen dann ganz oder teilweise
   mit einer oder mehreren aminofunktionellen Verbindungen B), wie primären und/oder sekundären Aminen und/oder Diaminen,
umsetzt. Die erfindungsgemäß verwendeten Polyurethane werden bevorzugt vor, während oder nach Schritt B) in Wasser dispergiert.

Besondere bevorzugt erfolgt im Schritt B) die Umsetzung mit einem Diamin oder mehreren Diaminen unter Kettenverlängerung. Dabei können zusätzlich monofunktionelle Amine als Kettenabbrecher zur Molekulargewichtssteuerung zugesetzt werden.

Als Komponente B) können insbesondere Amine verwendet werden, die keine ionischen bzw. ionogenen, wie anionisch hydrophilierende Gruppen aufweisen (im folgenden Komponente B1)) und es können Amine verwendet werden, die ionische bzw. ionogene, wie insbesondere anionisch hydrophilierenden Gruppen aufweisen (im folgenden Komponente B2)).

Bevorzugt wird im Schritt B) der Umsetzung des Prepolymers eine Mischung aus Komponente B1) und Komponente B2) zur Umsetzung gebracht. Durch die Verwendung der Komponente B1) kann eine hohe Molmasse aufgebaut werden, ohne dass die Viskosität des zuvor hergestellten isocyanatfunktionellen Präpolymers in einem Maße ansteigt, welches einer Verarbeitung entgegenstehen würde. Durch die Verwendung der Kombination der Komponenten B1) und B2) läßt sich eine optimale Balance zwischen Hydrophilie und Kettenlänge und damit ein angenehmes Hautgefühl einstellen.

Die erfindungsgemäß verwendeten Polyurethane weisen bevorzugt anionische Gruppen, bevorzugt Sulfonatgruppen auf. Diese anionischen Gruppen werden in die erfindungsgemäß verwendeten Polyurethanen über die in Schritt B) umgesetzte Aminkomponente B2) eingeführt. Die erfindungsgemäß verwendeten Polyurethane weisen gegebenenfalls zusätzlich nicht-ionische Komponenten zu Hydrophilierung auf. Besonders bevorzugt sind in den erfindungsgemäß verwendeten Polyurethanen zur Hydrophilierung ausschließlich Sulfonatgruppen enthalten, welche über entsprechende Diamine als Komponente B2) in das Polyurethan eingeführt werden.

Um eine gute Sedimentationsstabilität zu erreichen, liegt die zahlenmittlere Teilchengröße der speziellen Polyurethan-Dispersionen bevorzugt bei weniger als 750 nm, besonders bevorzugt bei weniger als 500 nm, bestimmt mittels Laserkorrelations-Spektroskopie nach Verdünnung mit entionisiertem Wasser (Gerät: Malvern Zetasizer 1000, Malver Inst. Limited).

Der Feststoffgehalt der Polyurethan-Dispersionen, welche zur Herstellung der dekorativen kosmetischen Zusammensetzung der Erfindung bevorzugt verwendet wird, beträgt im allgemeinen 10 bis 70, bevorzugt 30 bis 65, besonders bevorzugt 40 bis 60 Gew.-%. Die Feststoffgehalte werden ermittelt durch Erhitzen einer ausgewogenen Probe auf 125°C bis zur Gewichtskonstanz. Bei Gewichtskonstanz wird durch erneutes Auswiegen der Probe der Festkörpergehalt berechnet.

Bevorzugt weisen diese Polyurethan-Dispersionen weniger als 5 Gew.-%, besonders bevorzugt weniger als 0,2 Gew.-%, bezogen auf die Masse der Dispersionen, an ungebundenen organischen Aminen auf. Der Gehalt in den dekorativen kosmetischen Zusammensetzungen ist entsprechend noch geringer.

Geeignete Polyisocyanate der Komponente A1) sind insbesondere die dem Fachmann an sich bekannten aliphatischen, aromatischen oder cycloaliphatischen Polyisocyanate einer NCO-Funktionalität von größer oder gleich 2.

Beispiele solcher geeigneten Polyisocyanate sind 1,4-Butylendiisocyanat, 1,6-Hexamethylendiisocyanat (HDI), Isophorondiisocyanat (IPDI), 2,2,4- und/oder 2,4,4-Trimethylhexamethylendiisocyanat, die isomeren Bis-(4,4'-Isocyanatocyclohexyl)-methane oder deren Mischungen beliebigen Isomerengehalts, 1,4-Cyclohexylendiisocyanat, 4-Isocyanatomethyl-1,8-octandiisocyanat (Nonantriisocyanat), 1,4-Phenylendiisocyanat, 2,4- und/oder 2,6-Toluylendiisocyanat, 1,5-Naphthylendiisocyanat, 2,2'-und/oder 2,4'- und/oder 4,4'-Diphenylmethandiisocyanat, 1,3-und/oder 1,4-Bis-(2-isocyanato-prop-2-yl)-benzol (TMXDI), 1,3-Bis(isocyanatomethyl)benzol (XDI) sowie Alkyl-2,6-diisocyanatohexanoate (Lysindiisocyanate) mit C1-C8-Alkylgruppen.

Neben den vorstehend genannten Polyisocyanaten können auch modifizierte Diisocyanate die eine Funktionalität ≥ 2 aufweisen, mit Uretdion-, Isocyanurat-, Urethan-, Allophanat-, Biuret-, Iminooxadiazindion- oder Oxadiazintrionstruktur sowie Mischungen aus diesen anteilig eingesetzt werden.

Bevorzugt handelt es sich um Polyisocyanate oder Polyisocyanatgemische der vorstehend genannten Art mit ausschließlich aliphatisch oder cycloaliphatisch gebundenen Isocyanatgruppen oder Mischungen aus diesen und einer mittleren NCO-Funktionalität der Mischung von 2 bis 4, bevorzugt 2 bis 2,6 und besonders bevorzugt 2 bis 2,4, ganz besonders bevorzugt 2.

Besonders bevorzugt werden in A1) Hexamethylendiisocyanat, Isophorondiisocyanat oder die isomeren Bis-(4,4'-isocyanatocyclohexyl)methane sowie Mischungen der vorgenannten Diisocyanate eingesetzt.

In A2) werden polymere Polyole mit einem zahlenmittleren Molekulargewicht Mₙ von bevorzugt 400 bis 8000 g/mol, bevorzugter von 400 bis 6000 g/mol und besonders bevorzugt von 600 bis 3000 g/mol eingesetzt. Diese weisen bevorzugt eine OH-Funktionalität von 1,5 bis 6, besonders bevorzugt von 1,8 bis 3, ganz besonders bevorzugt von 1,9 bis 2,1 auf.

Der Ausdruck "polymere" Polyole bedeutet hier insbesondere, dass die genannten Polyole mindestens zwei, bevorzugter mindestens drei miteinander verbundene Wiederholungseinheiten aufweisen.

Solche polymeren Polyole sind die in der Polyurethanlacktechnologie an sich bekannten Polyesterpolyole, Polyacrylatpolyole, Polyurethanpolyole, Polycarbonat-polyole, Polyetherpolyole, Polyesterpolyacrylatpolyole, Polyurethanpolyacrylatpolyole, Polyurethanpolyesterpolyole, Polyurethanpolyetherpolyole, Polyurethanpolycarbonatpolyole und Polyesterpolycarbonatpolyole. Diese können in A2) einzeln oder in beliebigen Mischungen untereinander eingesetzt werden.

Die bevozugt verwendeten Polyesterpolyole sind die an sich bekannten Polykondensate aus Di- sowie gegebenenfalls Tri- und Tetraolen und Di- sowie gegebenenfalls Tri- und Tetracarbonsäuren oder Hydroxycarbonsäuren oder Lactonen. Anstelle der freien Polycarbonsäuren können auch die entsprechenden Polycarbonsäureanhydride oder entsprechende Polycarbonsäureester von niederen Alkoholen zur Herstellung der Polyester verwendet werden.

Beispiele für geeignete Diole sind Ethylenglykol, Butylenglykol, Diethylenglykol, Triethylenglykol, Polyalkylenglykole wie Polyethylenglykol, weiterhin 1,2-Propandiol, 1,3-Propandiol, Butandiol(1,3), Butandiol(1,4), Hexandiol(1,6) und Isomere, Neopentylglykol oder Hydroxypivalinsäureneopentylglykolester, wobei Hexandiol(1,6) und Isomere, Butandiol(1,4), Neopentylglykol und Hydroxypivalinsäureneopentylglykolester bevorzugt sind. Daneben können auch Polyole wie Trimethylolpropan, Glycerin, Erythrit, Pentaerythrit, Trimetylolbenzol oder Trishydroxyethylisocyanurat eingesetzt werden.

Als Dicarbonsäuren können Phthalsäure, Isophthalsäure, Terephthalsäure, Tetrahydrophthalsäure, Hexahydrophthalsäure, Cyclohexandicarbonsäure, Adipinsäure, Azelainsäure, Sebacinsäure, Glutarsäure, Tetrachlorphthalsäure, Maleinsäure, Fumarsäure, Itaconsäure, Malonsäure, Korksäure, 2-Methylbernsteinsäure, 3,3-Diethylglutarsäure und/oder 2,2-Dimethylbernsteinsäure eingesetzt werden. Als Säurequelle können auch die entsprechenden Anhydride verwendet werden.

Sofern die mittlere Funktionalität des zu veresternden Polyols > als 2 ist, können zusätzlich auch Monocarbonsäuren, wie Benzoesäure und Hexancarbonsäure mit verwendet werden.

Bevorzugte Säuren sind aliphatische oder aromatische Säuren der vorstehend genannten Art. Besonders bevorzugt sind Adipinsäure, Isophthalsäure und Phthalsäure.

Hydroxycarbonsäuren, die als Reaktionsteilnehmer bei der Herstellung eines Polyesterpolyols mit endständigen Hydroxylgruppen mitverwendet werden können, sind beispielsweise Hydroxycapronsäure, Hydroxybuttersäure, Hydroxydecansäure, Hydroxystearinsäure und dergleichen. Geeignete Lactone sind Caprolacton, Butyrolacton und Homologe. Bevorzugt ist Caprolacton.

Erfindungsgemäß besonders bevorzugt sind als Komponente A2) zur Herstellung der Polyurethane, Polyesterpolyole mit einem zahlenmittleren Molekulargewicht von 600 bis 3000 g/mol, insbesondere aliphatische Polyesterpolyole auf Basis aliphatischer Carbonsäuren und aliphatischer Polyole, insbesondere auf Basis von Adipinsäure und aliphatischen Alkoholen, wie Hexandiol und/oder Neopentylglykol.

Ebenfalls können als Komponente A2) hydroxylgruppenaufweisende Polycarbonate, bevorzugt Polycarbonatdiole, mit zahlenmittleren Molekulargewichten Mₙ von bevorzugt 400 bis 8000 g/mol, bevorzugt 600 bis 3000 g/mol eingesetzt werden. Diese sind durch Reaktion von Kohlensäurederivaten, wie Diphenylcarbonat, Dimethylcarbonat oder Phosgen, mit Polyolen, bevorzugt Diolen, erhältlich.

Beispiele derartiger Diole sind Ethylenglykol, 1,2- und 1,3-Propandiol, 1,3- und 1,4-Butandiol, 1,6-Hexandiol, 1,8-Octandiol, Neopentylglykol, 1,4-Bishydroxymethylcyclohexan, 2-Methyl-1,3-propandiol, 2,2,4-Trimethylpentandiol-1,3, Dipropylenglykol, Polypropylenglykole, Dibutylenglykol, Polybutylenglykole, Bisphenol A und lactonmodifizierte Diole der vorstehend genannten Art.

Bevorzugt enthält die Diolkomponente 40 bis 100 Gew.-% Hexandiol, bevorzugt sind 1,6-Hexandiol und/oder Hexandiolderivate. Solche Hexandiolderivate basieren auf Hexandiol und weisen neben endständigen OH-Gruppen Ester- oder Ethergruppen auf. Solche Derivate sind durch Reaktion von Hexandiol mit überschüssigem Caprolacton oder durch Veretherung von Hexandiol mit sich selbst zum Di- oder Trihexylenglykol erhältlich.

Statt oder zusätzlich zu reinen Polycarbonatdiolen können auch Polyether-Polycarbonatdiole in A2) eingesetzt werden.

Hydroxylgruppen aufweisende Polycarbonate sind bevorzugt linear gebaut.

Ebenfalls können als Komponente A2) Polyetherpolyole eingesetzt werden.

Besonders geeignet sind beispielsweise die in der Polyurethanchemie an sich bekannten Polytetramethylenglykolpolyether wie sie durch Polymerisation von Tetrahydrofuran mittels kationischer Ringöffnung erhältlich sind.

Ebenfalls geeignete Polyetherpolyole sind die an sich bekannten Additionsprodukte von Styroloxid, Ethylenoxid, Propylenoxid, Butylenoxid und/oder Epichlorhydrin an di-oder polyfunktionelle Startermoleküle. So sind insbesondere Polyalkylenglykole, wie Polyethylen-, Polypropylen- und/oder Polybutylenglykole anwendbar, insbesondere mit den oben genannten bevorzugten Molekulargewichten.

Als geeignete Startermoleküle können alle dem Stand der Technik nach bekannten Verbindungen eingesetzt werden, wie zum Beispiel Wasser, Butyldiglykol, Glycerin, Diethylenglykol, Trimethyolpropan, Propylenglykol, Sorbit, Ethylendiamin, Triethanolamin, 1,4-Butandiol.

Besonders bevorzugte Komponenten in A2) sind Polytetramethylenglykolpolyether und Polycarbonat-Polyole bzw. deren Mischungen und besonders bevorzugt sind Polytetramethylenglykolpolyether.

In bevorzugten Ausführungsformen der Erfindung handelt es sich bei Komponente A2) demnach um:
- Mischungen, enthaltend wenigstens ein Polyether-Polyol und wenigstens ein Polycarbonat-Polyof,
- Mischungen, enthaltend mehr als ein Polyether-Polyol, bzw. ein Gemisch mehrerer Polyether-Polyole mit unterschiedlichen Molekulargewichten, wobei es sich insbesondere um Poly(tetramethylenglykol)polyetherpolyole (wie (HO-(CH₂-CH₂-CH₂-CH₂-O)_{X}-H) handelt,
- Mischungen, enthaltend mehr als ein Polyether-Polyol, und wenigstens ein Polycarbonat-Polyol, sowie
- besonders bevorzugt Polyesterpolyole mit einem zahlenmittleren Molekulargewicht von 600 bis 3000 g/mol, insbesondere aliphatische Polyesterpolyole auf Basis aliphatischer Carbonsäuren und aliphatischer Polyole, insbesondere auf Basis von Adipinsäure und aliphatischen Alkoholen, wie Hexandiol
   und/oder Neopentylglykol,
wobei die Komponente A) definitionsgemäß im Wesentlichen weder ionische noch ionogen Gruppen aufweist.

Als Komponente A3) können wahlweise Polyole, insbesondere nicht-polymere Polyole, des bevorzugt genannten Molekulargewichtsbereichs von 62 bis 399 mol/g mit bis zu 20 Kohlenstoffatomen, wie Ethylenglykol, Diethylenglykol, Triethylenglykol, 1,2-Propandiol, 1,3-Propandiol, 1,4-Butandiol, 1,3-Butylenglykol, Cyclohexandiol, 1,4-Cyclohexandimethanol, 1,6-Hexandiol, Neopentylglykol, Hydrochinondihydroxyethylether, Bisphenol A (2,2-Bis(4-hydroxyphenyl)propan), hydriertes Bisphenol A (2,2-Bis(4-hydroxycyclohexyl)propan), Trimethylolpropan, Trimethylolethan, Glycerin, Pentaerythrit sowie deren beliebige Mischungen untereinander eingesetzt werden.

Geeignet sind auch Esterdiole des genannten Molekulargewichtsbereichs wie α-Hydroxybutyl-ε-hydroxy-capronsäureester, ω-Hydroxyhexyl-γ-hydroxybuttersäureester, Adipinsäure-(β-hydroxyethyl)ester oder Terephthalsäurebis(β-hydroxyethyl)-ester.

Ferner können als Komponente A3) auch monofunktionelle isocyanatreaktive Hydroxyl-gruppenhaltige Verbindungen eingesetzt werden. Beispiele solcher monofunktionellen Verbindungen sind Ethanol, n-Butanol, Ethylenglykolmonobutylether, Diethylenglykolmonomethylether, Diethylenglykolmonobutylether, Propylenglykolmonomethylether, Dipropylenglykol-monomethylether, Tripropylenglykolmonomethylether, Dipropylenglykolmono-propylether, Propylenglykolmonobutylether, Dipropylenglykolmonobutylether, Tripropylenglykolmonobutylether, 2-Ethylhexanol, 1-Octanol, 1-Dodecanol, 1-Hexadecanol.

In einer bevorzugten Ausführungsform der Erfindung enthält dass erfindungsgemäß verwendete Polyurethan weniger als etwa 10 Gew.-% der Komponente A3), bevorzugt weniger als 5 Gew.-% der Komponente A3), jeweils bezogen auf die Gesamtmasse des Polyurethans, noch bevorzugter wird Komponente A3) zur Herstellung des Polyurethans nicht verwendet.

Als Komponente A4) werden zur Herstellung der erfindungsgemäß verwendeten Polyurethane gegebenenfalls ein oder mehrere insbesondere isocyanatreaktive nichtionische Hydrophilierungsmittel verwendet. Die als Komponente A4) verwendeten Hydrophilierungsmittel sind insbesondere von den Komponenten A2) und A3) verschieden.

Geeignete nichtionisch hydrophilierende Verbindungen als Komponente A4) sind z.B. Polyoxyalkylenether, welche über isocyanatreaktive Gruppen, wie Hydroxy-, Amino-oder Thiolgruppen verfügen. Bevorzugt sind monohydroxyfunktionelle, im statistischen Mittel 5 bis 70, bevorzugt 7 bis 55 Ethylenoxideinheiten pro Molekül aufweisenden Polyalkylenoxidpolyetheralkohole, wie sie in an sich bekannter Weise durch Alkoxylierung geeigneter Startermoleküle zugänglich sind (z.B. in Ullmanns Encyclopädie der technischen Chemie, 4. Auflage, Band 19, Verlag Chemie, Weinheim S. 31-38). Diese sind entweder reine Polyethylenoxidether oder gemischte Polyalkylenoxidether, wobei sie mindestens 30 mol-%, bevorzugt mindestens 40 mol-% bezogen auf alle enthaltenen Alkylenoxideinheiten an Ethylenoxideinheiten enthalten.

Besonders bevorzugte nichtionische Verbindungen sind monofunktionelle gemischte Polyalkylenoxidpolyether, die 40 bis 100 mol-% Ethylenoxid- und 0 bis 60 mol-% Propylenoxideinheiten aufweisen.

Geeignete Startermoleküle für solche nichtionischen Hydrophilierungsmittel sind insbesondere gesättigte Monoalkohole wie Methanol, Ethanol, n-Propanol, Isopropanol, n-Butanol, Isobutanol, sec-Butanol, die isomeren Pentanole, Hexanol, Octanole und Nonanole, n-Decanol, n-Dodecanol, n-Tetradecanol, n-Hexadecanol, n-Octadecanol, Cyclohexanol, die isomeren Methylcyclohexanole oder Hydroxymethylcyclohexan, 3-Ethyl-3-hydroxymethyloxetan oder Tetrahydrofurfurylalkohol, Diethylenglykolmonoalkylether, wie beispielsweise Diethylenglykolmonobutylether, ungesättigte Alkohole wie Allylalkohol, 1,1-Dimethylallylalkohol oder Oleinalkohol, aromatische Alkohole wie Phenol, die isomeren Kresole oder Methoxyphenole, araliphatische Alkohole wie Benzylalkohol, Anisalkohol oder Zimtalkohol, sekundäre Monoamine wie Dimethylamin, Diethylamin, Dipropylamin, Diisopropylamin, Dibutylamin, Bis-(2-ethylhexyl)-amin, N-Methyl- und N-Ethylcyclohexylamin oder Dicyclohexylamin sowie heterocyclische sekundäre Amine wie Morpholin, Pyrrolidin, Piperidin oder 1 H-Pyrazol. Bevorzugte Startermoleküle sind gesättigte Monoalkohole der vorstehend genannten Art. Besonders bevorzugt werden Diethylenglykolmonobutylether oder n-Butanol als Startermoleküle verwendet.

Für die Alkoxylierungsreaktion geeignete Alkylenoxide sind insbesondere Ethylenoxid und Propylenoxid, die in beliebiger Reihenfolge oder auch im Gemisch bei der Alkoxylierungsreaktion eingesetzt werden können.

Die Komponente B) wird bevorzugt aus primären oder sekundären Amin und/oder Diaminen ausgewählt. Sie umfasst insbesondere Diamine.

Als Komponente B) können insbesondere Amine verwendet werden, die keine ionischen bzw. ionogenen, wie anionisch hydrophilierenden Gruppen aufweisen (im folgenden Komponente B1)), und es können Amine verwendet werden, die ionische bzw. ionogene, wie insbesondere anionisch hydrophilierenden Gruppen aufweisen (im folgenden Komponente B2)). Bevorzugt wird im Schritt B) der Umsetzung des Prepolymers eine Mischung aus der Komponente B1) und der Komponente B2) zur Umsetzung gebracht.

Beispielsweise können als Komponente B1) organische Di- oder Polyamine wie beispielsweise 1,2-Ethylendiamin, 1,2- und 1,3-Diaminopropan, 1,4-Diaminobutan, 1,6-Diaminohexan, Isophorondiamin, Isomerengemisch von 2,2,4- und 2,4,4-Trimethylhexamethylendiamin, 2-Methylpentamethylendiamin, Diethylentriamin, 4,4-Diaminodicyclohexylmethan, Hydrazinhydrat, und/oder Dimethylethylendiamin eingesetzt werden.

Darüber hinaus können als Komponente B1) auch Verbindungen, die neben einer primären Aminogruppe auch sekundäre Aminogruppen oder neben einer Aminogruppe (primär oder sekundär) auch OH-Gruppen aufweisen, eingesetzt werden. Beispiele hierfür sind primäre/sekundäre Amine, wie Diethanolamin, 3-Amino-1-methylaminopropan, 3-Amino-1-ethylaminopropan, 3-Amino-1-cyclohexylaminopro-pan, 3-Amino-1- methylaminobutan, Alkanolamine wie N-Aminoethylethanolamin, Ethanolamin, 3-Aminopropanol, Neopentanolamin.

Ferner können als Komponente B1) auch monofunktionelle isocyanatreaktive Aminverbindungen eingesetzt werden, wie beispielsweise Methylamin, Ethylamin, Propylamin, Butylamin, Octylamin, Laurylamin, Stearylamin, Isononyloxypropylamin, Dimethylamin, Diethylamin, Dipropylamin, Dibutylamin, N-Methylaminopropylamin, Diethyl(methyl)aminopropylamin, Morpholin, Piperidin, bzw. geeignete substituierte Derivate davon, Amidamine aus diprimären Aminen und Monocarbonsäuren, Monoketim von diprimären Aminen, primär/tertiäre Amine, wie N,N-Dimethylaminopropylamin.

Bevorzugt werden als Komponente B1) 1,2-Ethylendiamin, Bis(4-aminocyclohexyl)methan, 1,4-Diaminobutan, Isophorondiamin, Ethanolamin, Diethanolamin und Diethylentriamin eingesetzt.

Besonders bevorzugt umfasst die Komponente B) mindestens eine Komponente B2). Geeignete anionisch hydrophilierende Verbindungen als Komponente B2) enthalten bevorzugt eine Sulfonsäure- oder Sulfonatgruppe, besonders bevorzugt eine Natriumsulfonatgruppe. Geeignete anionisch hydrophilierende Verbindungen als Komponente B2) sind insbesondere die Alkalimetallsalze der Mono- und Diaminosulfonsäuren. Beispiele solcher anionischen Hydrophilierungsmittel sind Salze der 2-(2-Aminoethyiamino)ethansulfonsäure, Ethylendiamin-propyl- oder -butylsulfonsäure, 1,2- oder 1,3-Propylendiamin-β-ethylsulfonsäure oder Taurin. Weiterhin kann das Salz der Cyclohexylaminopropansulfonsäure (CAPS) aus WO-A 01/88006 als anionisches Hydrophilierungsmittel verwendet werden.

Besonders bevorzugte anionische Hydrophilierungsmittel B2) sind solche, die - Sulfonatgruppen als ionische Gruppen und zwei Aminogruppen enthalten, wie die Salze der 2-(2-Aminoethylamino)ethylsulfonsäure und 1,3-Propylendiamin-βethylsulfonsäure.

Besonders bevorzugt enthaltend die erfindungsgemäß verwendeten Polyurethane mindestens eine Sulfonatgruppe.

Gegebenenfalls kann die anionische Gruppe in der Komponente B2) auch eine Carboxylat bzw. Carbonsäuregruppe sein. Die Komponente B2) wird dann bevorzugt aus Diaminocarbonsäuren ausgewählt. Diese Ausführungsform ist allerdings weniger bevorzugt, da Carbonsäure-basierende Komponenten B2) in höheren Konzentrationen eingesetzt werden müssen.

Zur Hydrophilierung können auch Mischungen aus anionischen Hydrophilierungsmitteln B2) und nichtionischen Hydrophilierungsmitteln A4) verwendet werden.

In einer bevorzugten Ausführungsform zur Herstellung der speziellen Polyurethan-Dispersionen werden die Komponenten A1) bis A4) und B1) bis B2) in den folgenden Mengen eingesetzt, wobei sich die Einzelmengen stets zu 100 Gew.-% addieren:
5 bis 40 Gew.-% Komponente A1),
55 bis 90 Gew.-% A2),
0,5 bis 20 Gew.-% Summe der Komponenten A3) und/oder B1)
0,1 bis 25 Gew.-% Summe der Komponenten A4) und/oder B2), wobei bezogen auf die Gesamtmengen der Komponenten A1) bis A4) und B1) bis B2) besonders bevorzugt 0,1 bis 5 Gew.-% an anionischen bzw. potentiell anionischen Hydrophilierungsmittein B2) verwendet werden.

In einer besonders bevorzugten Ausführungsform zur Herstellung der speziellen Polyurethan-Dispersionen werden die Komponenten A1) bis A4) und B1) bis B2) in den folgenden Mengen eingesetzt, wobei sich die Einzelmengen stets zu 100 Gew.-% aufaddieren:
5 bis 35 Gew.-% Komponente A1),
60 bis 90 Gew.-% A2),
0,5 bis 15 Gew.-% Summe der Komponenten A3) und/oder B1)
0,1 bis 15 Gew.-% Summe der Komponenten Komponente A4) und/oder B2), wobei bezogen auf die Gesamtmengen der Komponenten A1) bis A4) und B1) bis B2) besonders bevorzugt 0,2 bis 4 Gew.-% an anionischen bzw. potentiell anionischen Hydrophilierungsmitteln B2) verwendet werden.

In einer ganz besonders bevorzugten Ausführungsform zur Herstellung der speziellen Polyurethan-Dispersionen werden die Komponenten A1) bis A4) und B1) bis B2) in den folgenden Mengen eingesetzt, wobei sich die Einzelmengen stets zu 100 Gew.-% aufaddieren:
10 bis 30 Gew.-% Komponente A1),
65 bis 85 Gew.-% A2),
0,5 bis 14 Gew.-% Summe der Komponenten A3) und/oder B1)
0,1 bis 13,5 Gew.-% Summe der Komponenten A4) und/oder B2), wobei bezogen auf die Gesamtmengen der Komponenten A1) bis A4) und B1) bis B2) besonders bevorzugt 0,5 bis 3,0 Gew.-% an anionischen bzw. potentiell anionischen Hydrophilierungsmitteln aus B2) verwendet werden.

Die Herstellung der Polyurethan-Dispersionen kann in einer oder mehreren Stufe/-n in homogener oder bei mehrstufiger Umsetzung, teilweise in disperser Phase durchgeführt werden. Nach vollständig oder teilweise durchgeführter Polyaddition aus A1) bis A4) erfolgt bevorzugt ein Dispergier-, Emulgier- oder Lösungsschritt. Im Anschluss erfolgt gegebenenfalls eine weitere Polyaddition oder Modifikation in disperser Phase.

Dabei können alle aus dem Stand der Technik bekannten Verfahren wie z. B. Prepolymer-Mischverfahren, Acetonverfahren oder Schmelzdispergierverfahren verwendet werden. Bevorzugt wird nach das Aceton-Verfahren angewandt.

Für die Herstellung nach dem Aceton-Verfahren werden üblicherweise die Bestandteile A2) bis A4) und die Polyisocyanatkomponente A1) zur Herstellung eines isocyanatfunktionellen Polyurethan-Prepolymers ganz oder teilweise vorgelegt und gegebenenfalls mit einem mit Wasser mischbaren aber gegenüber Isocyanatgruppen inerten Lösungsmittel verdünnt und auf Temperaturen im Bereich von 50 bis 120°C aufgeheizt. Zur Beschleunigung der Isocyanatadditionsreaktion können die in der Polyurethan-Chemie bekannten Katalysatoren eingesetzt werden.

Geeignete Lösungsmittel sind die üblichen aliphatischen, ketofunktionellen Lösemittel wie Aceton, 2-Butanon, die nicht nur zu Beginn der Herstellung, sondern gegebenenfalls in Teilen auch später zugegeben werden können. Bevorzugt sind Aceton und 2-Butanon, besonders bevorzugt ist Aceton. Auch die Zugabe anderer Lösemittel ohne isocyanatreaktive Gruppen ist möglich, jedoch nicht bevorzugt.

Anschließend werden die gegebenenfalls zu Beginn der Reaktion noch nicht zugegebenen Bestandteile von A1) bis A4) zudosiert.

Bei der Herstellung des Polyurethan-Prepolymeren aus A1) bis A4) beträgt das Stoffmengenverhältnis von Isocyanat-Gruppen zu isocyanatreaktiven Gruppen im allgemeinen 1,05 bis 3,5, bevorzugt 1,1 bis 3,0, besonders bevorzugt 1,1 bis 2,5.

Die Umsetzung der Komponenten A1) bis A4) zum Prepolymer erfolgt teilweise oder vollständig, bevorzugt aber vollständig. Es werden so Polyurethan-Prepolymere, die freie Isocyanatgruppen enthalten, in Substanz oder in Lösung erhalten.

Im Neutralisationsschritt zur teilweisen oder vollständigen Überführung potentiell anionischer Gruppen in anionische Gruppen werden Basen wie tertiäre Amine, z.B. Trialkylamine mit 1 bis 12, bevorzugt 1 bis 6 C-Atomen, besonders bevorzugt 2 bis 3 C-Atomen in jedem Alkylrest oder ganz besonders bevorzugt Alkalimetallbasen wie die entsprechenden Hydroxide eingesetzt.

Die Verwendung von organische Aminen ist nicht bevorzugt.

Als Neutralisationsmittel werden bevorzugt anorganische Basen, wie wässrige Ammoniaklösung oder Natrium- bzw. Kaliumhydroxid einsetzbar.

Bevorzugt sind Natriumhydroxid und Kaliumhydroxid,

Die Stoffmenge der Basen beträgt 50 und 125 mol%, bevorzugt zwischen 70 und 100 mol% der Stoffmenge der zu neutralisierenden Säuregruppen. Die Neutralisation kann auch gleichzeitig mit der Dispergierung erfolgen, in dem das Dispergierwasser bereits das Neutralisationsmittel enthält.

Im Anschluss wird in einem weiteren Verfahrensschritt, falls noch nicht oder nur teilweise geschehen, das erhaltene Prepolymer mit Hilfe von aliphatischen Ketonen wie Aceton oder 2-Butanon gelöst.

Die Umsetzung der Komponenten A1) bis A4) zum Prepolymer erfolgt teilweise oder vollständig, bevorzugt aber vollständig. Es werden so Polyurethan-Prepolymere, die freie Isocyanatgruppen enthalten, in Substanz oder in Lösung erhalten.

Bei der Kettenverlängerung in Stufe B) werden NH₂- und/oder NH-funktionelle Komponenten mit den noch verbliebenen Isocyanatgruppen des Prepolymers umgesetzt. Bevorzugt wird die Kettenverlängerung/-terminierung vor der Dispergierung in Wasser durchgeführt.

Geeignete Komponenten B) zur Kettenverlängerung sind insbesondere organische Di- oder Polyamine B1) wie beispielsweise Ethylendiamin, 1,2- und 1,3-Diaminopropan, 1,4-Diaminobutan, 1,6-Diaminohexan, Isophorondiamin, Isomerengemisch von 2,2,4- und 2,4,4-Trimethylhexamethylendiamin, 2-Methylpentamethylendiamin, Diethylentriamin, Diaminodicyclohexylmethan und/oder Dimethylethylendiamin.

Darüber hinaus können auch Verbindungen B1), die neben einer primären Aminogruppe auch sekundäre Aminogruppen oder neben einer Aminogruppe (primär oder sekundär) auch OH-Gruppen aufweisen, eingesetzt werden. Beispiele hierfür sind primäre/sekundäre Amine, wie Diethanolamin, 3-Amino-1-methylaminopropan, 3-Amino-1-ethylaminopropan, 3-Amino-1-cycloheoylaminopropan, 3-Amino-1-methylaminobutan, Alkanolamine wie N-Aminoethylethanolamin, Ethanolamin, 3-Aminopropanol, Neopentanolamin zur Kettenverlängerung bzw. -terminierung eingesetzt werden.

Zur Kettenterminierung werden üblicherweise Amine B1) mit einer gegenüber Isocyanaten reaktiven Gruppe wie Methylamin, Ethylamin, Propylamin, Butylamin, Octylamin, Laurylamin, Stearylamin, Isononyloxypropylamin, Dimethylamin, Diethylamin, Dipropylamin, Dibutylamin, N-Methylaminopropylamin, Diethyl(methyl)aminopropylamin, Morpholin, Piperidin, bzw. geeignete substituierte Derivate davon, Amidamine aus diprimären Aminen und Monocarbonsäuren, Monoketim von diprimären Aminen, primär/tertiäre Amine, wie N,N-Dimethylaminopropylamin verwendet.

Werden zur Kettenverlängerung anionische Hydrophilierungsmittel entsprechend der Definition B2) mit NH₂- oder NH-Gruppen eingesetzt, erfolgt die Kettenverlängerung der Prepolymere bevorzugt vor der Dispergierung.

Der Kettenverlängerungsgrad, also das Äquivalentverhältnis von NCO-reaktiven Gruppen der zur Kettenverlängerung und Kettenterminierung eingesetzten Verbindungen zu freien NCO-Gruppen des Prepolymers, liegt im allgemeinen zwischen 40 und 150 %, bevorzugt zwischen 50 und 110 %, besonders bevorzugt zwischen 60 und 100 %.

Die aminischen Komponenten B1) und B2), können gegebenenfalls in wasser- oder lösemittelverdünnter Form im erfindungsgemäßen Verfahren einzeln oder in Mischungen eingesetzt werden, wobei grundsätzlich jede Reihenfolge der Zugabe möglich ist.

Wenn Wasser oder organische Lösemittel als Verdünnungsmittel mit verwendet werden, so beträgt der Verdünnungsmittelgehalt in der in B) eingesetzten Komponente zur Kettenverlängerung bevorzugt 40 bis 95 Gew.-%.

Die Dispergierung erfolgt bevorzugt im Anschluss an die Kettenverlängerung. Dazu wird das gelöste und kettenverlängerte Polyurethanpolymer gegebenenfalls unter starker Scherung, wie z.B. starkem Rühren, entweder in das Dispergierwasser eingetragen oder es wird umgekehrt das Dispergierwasser zu den kettenverlängerte Polyurethanpolymerlösungen gerührt. Bevorzugt wird das Wasser in das gelöste kettenverlängerte Polyurethanpolymer gegeben.

Das in den Dispersionen nach dem Dispergierschritt noch enthaltene Lösemittel wird üblicherweise anschließend destillativ entfernt. Eine Entfernung bereits während der Dispergierung ist ebenfalls möglich.

Der Restgehalt an organischen Lösemitteln in den so hergestellten Polyurethan-Dispersionen beträgt typischerweise weniger als 10 Gew.-%, bevorzugt weniger als 3 Gew.-%, bezogen auf die gesamte Dispersion.

Der pH-Wert der erfindungsgemäß verwendeten wässrigen Polyurethan-Dispersionen beträgt typischerweise weniger als 8,0, bevorzugt weniger als 7,5 und liegt besonders bevorzugt zwischen 5,5 und 7,5.

Die erfindungsgemäße dekorative kosmetische Zusammensetzung enthält bevorzugt 0,1 bis 20 Gewichts-% des oben beschriebenen Polyurethans und insbesondere 0,5 bis 10 Gewichts-% jeweils bezogen auf das Gesamtgewicht der Zusammensetzung.

Die dekorative kosmetische Zusammensetzung der Erfindung dient der dekorativen, insbesondere farblichen oder effektgebenden Gestaltung der menschlichen Haut, Schleimhaut, Semi-Schleimhaut und des Haars, insbesondere der Augenlider und der Augenbrauen (im allgemeinen nicht des Kopfhaars). Der dekorative, d.h. Farbeffekt oder sonstige Effekt (Glitzereffekt, Metallic-Effekt usw.) wird durch mindestens einen effektgebenden, insbesondere farb- und/oder effektgebenden Bestandteil erzielt. Die erfindungsgemäße dekorative Zusammensetzung kann beispielsweise ein Gesichts-Make-up (Foundation), eine getönte (Tages)Creme, ein Blush, ein Rouge, eine Wimperntusche, ein Eyeliner, ein Kajal, ein Lidschatten, ein Lippenstift, ein Lip Gloss sein. Die erfindungsgemäße dekorative Zusammensetzung schließt solche zum Auftrag auf Nägel, wie Nagellack im Allgemeinen nicht ein. Ein Kennzeichen der dekorative kosmetischen Zusammensetzungen ist im Allgemeinen, dass es sich um sogenannte "leave on"-Produkte handelt, die nach dem Auftrag wenigstens zum Teil auf der Haut oder dem Haar verbleiben.

Die erfindungsgemäße dekorative kosmetische Zusammensetzung kann insbesondere fest, flüssig oder halbfest sein. Die Zusammensetzung kann beispielsweise in der Form von Öl-in-Wasser-, Wasser-in-Öl-, Wasser-in-Silikonöl-, Silikonöl-in-Wasser-, Öl-in-Wasser-in-Öl-, Wasser-in-Öl-in-Wasser- oder Feststoff-Emulsionen (Emulsion, welche durch Feststoffe stabilisiert sind wie beispielweise Pickering-Emulsionen) vorliegen. Die erfindungsgemäße Formulierung kann ferner mit einem Treibgas aufgeschäumt werden. Die erfindungsgemäße Formulierung kann weiterhin in der Form von "loose powder" (loses Pulver), Kompaktpulver, Schaum (so genannten Mousse), Stiften oder in der Form der oben genannten flüssigen oder viskosen Emulsionen vorliegen.

Die erfindungsgemäße Zusammensetzung enthält mindestens einen effektgebenden Bestandteil. Der genannten Bestandteil kann insbesondere farbgebend sein aber auch sonstige andere Effekte bereitstellen, wie Glitzer- und/oder Metalliceffekte. Bevorzugt enthält die erfindungsgemäße Zusammensetzung mindestens einen Farbstoff, der bevorzugt aus der Gruppe von lipophilen Farbstoffen, hydrophilen Farbstoffen, Pigmenten und Perlmutt ausgewählt wird. Erfindungsgemäß besonders vorteilhaft ist die Konzentration von Farbstoffen 0,01 bis 40 Gewichts-%, besonders vorteilhaft 1,0 bis 30 Gewichts-%, ganz besonders vorteilhaft von 2,0 bis 25 Gewichts-%, jeweils bezogen auf das Gesamtgewicht der Zusammensetzung.

Beispielweise können lipophile Farbstoffe verwendet werden, wie Sudan I (gelb), Sudan II (orange), Sudan III (rot), Sudan IV (Scharlachrot), DC Red 17, DC Green 6, β-Carotin, Sojaöl, DC Yellow 11, DC Violet 2, DC Orange 5 und DC Yellow 10.

Die Pigmente können prinzipiell alle anorganischen oder organischen Pigmente sein, die in kosmetischen oder dermatologischen Zusammensetzung verwendet werden. Die erfindungsgemäßen verwendeten Pigmente können beispielsweise weiß oder farbig sein, sie können umgehüllt bzw. beschichtet sein mit einem hydrophoben Behandlungsmittel oder nicht beschichtet sein.

Vorteilhaft werden die Pigmente gewählt aus der Gruppe der Metalloxide, wie die Oxide von Eisen (insbesondere die Oxide von gelber, roter, brauner, schwarzer Farbe), Titandioxid, Zinkoxid, Ceroxid, Zirconiumoxid, Chromoxid; Manganviolett, Ultramarinblau, Preussisch Blau, Ultramarin und Eisenblau, Bismutoxidchlorid, Perlmutt, mit Titan oder Bismutoxidchlorid überzogene Glimmer-Pigmente, farbige Perlglanzpigmente, beispielsweise Titan-Glimmer-Pigmente mit Eisenoxiden, Titan-Glimmer-Pigmente insbesondere mit Eisenblau oder Chromoxid, Titan-Glimmer-Pigmente mit einem organischen Pigment vom vorgenannten Typ, sowie Perlglanzpigmente auf der Basis von Bismutoxidchlorid, Russ, die Pigmente vom Typ D & C und die Lacke auf der Basis von Cochenillerot, Barium, Strontium, Calcium und Aluminium und deren Gemische.

Besonders vorteilhaft werden die Pigmente von Eisenoxiden oder Titandioxid verwendet.

Für eine bessere Benetzbarkeit der Pigmente durch die Öle der Fettphase wird die Oberfläche der Pigmente bevorzugt mit einem hydrophoben Behandlungsmittel behandelt. Das hydrophobe Behandlungsmittel wird bevorzugt ausgewählt aus der Gruppe der Silicone, wie Methicone, Dimethicone, Perfluoralkylsilane; Fettsäuren wie Stearinsäure; Metallseifen, wie Aluminiumdimyristat, dem Aluminiumsalz von hydriertem Talgglutamat, Perfluoralkylphosphaten, Perfluoralkylsilanen, Perfluoralkylsilazanen, Hexafluorpropylenpolyoxiden, Polyorganosiloxanen, die Perfluoralkylperfluorpo-Iyethergruppen enthalten, Aminosäuren; N-acylierten Aminosäuren oder deren Salzen; Lecithin, Isopropyltriisostearyltitanat und deren Gemischen ausgewählt sein. Die N-acylierten Aminosäuren können eine Acylgruppe mit 8 bis 22 Kohlenstoffatomen enthalten, beispielsweise 2-Ethylhexanoyl, Caproyl, Lauroyl, Myristoyl, Palmitoyl, Stearoyl oder Cocoyl. Die Salze dieser Verbindungen können Aluminiumsalze, Magnesiumsalze, Calciumsalze, Zirkoniumsalze, Zinnsalze, Natriumsalze oder Kaliumsalze sein. Bei der Aminosäure kann es sich beispielsweise um Lysin, Glutaminsäure oder Alanin handeln.

Die erfindungsgemäßen dekorativen kosmetischen Zusammensetzungen können einen oder mehrere Emulgatoren bzw. oberflächenaktive Mittel enthalten.

So enthalten erfindungsgemäße Öl-in-Wasser-Emulsionen (O/W) bevorzugt mindestens einen Emulgator mit einem HLB-Wert > 7 und gegebenenfalls einen Coemulgato r.

Vorteilhaft werden die folgenden nichtionischen Emulgatoren eingesetzt :
- a) Partialfettsäureester und Fettsäureester mehrwertiger Alkohole und deren ethoxylierte Derivate (z. B. Glycerylmonostearat, Sorbitanstearat, Glyceryl-Stearyl-Citrat, Sucrose-Stearat)
- b) ethoxylierte Fettalkohole und Fettsäuren.

Besonders vorteilhafte nichtionische O/W-Emulgatoren sind ethoxylierte Fettalkohole oder Fettsäuren, bevorzugt PEG-100-Stearat, PEG-40-Stearat, Ceteareth-20, Ceteth-20, Steareth-20, Ceteareth-12, Ceteth-12, Steareth-12, sowie Ester aus Mono-, Oligo- oder Polysacchariden mit Fettsäuren, bevorzugt Cetearylglucosid, Methylglucosedistearat.

Vorteilhafte anionische Emulgatoren sind Seifen (z. B. Natrium- oder TriethanolaminSalze der Stearin- oder Palmitinsäure) sowie Ester der Zitronensäure wie Glycerylstearatcitrat.

Als geeignete Coemulgatoren für die erfindungsgemässen O/W-Emulsionen können Fettalkohole mit 8 bis 30 Kohlenstoffatomen, Monoglycerinester gesättigter oder ungesättigter, verzweigter oder unverzweigter Alkancarbonsäuren mit einer Kettenlänge von 8 bis 24 Kohlenstoffatomen, insbesondere 12 bis 18 Kohlenstoffatomen, Propylenglycolester gesättigter oder ungesättigter, verzweigter oder unverzweigter Alkancarbonsäuren mit einer Kettenlänge von 8 bis 24 Kohlenstoffatomen, insbesondere 12 bis 18 Kohlenstoffatomen, sowie Sorbitanester gesättigter oder ungesättigter, verzweigter oder unverzweigter Alkancarbonsäuren mit einer Kettenlänge von 8 bis 24 Kohlenstoffatomen, insbesondere 12 bis 18 Kohlenstoffatomen, eingesetzt werden.

Besonders vorteilhafte Coemulgatoren sind Glycerylmonostearat, Glycerylmonooleat, Diglycerylmonostearat, Sorbitanmonoisostearat, Saccharosedistearat, Cetylalkohol, Stearylalkohol, Behenylalkohol, Isobehenylalkohol und Polyethylenglycol(2)stearylether (Steareth-2).

Es kann im Sinn der vorliegenden Erfindung vorteilhaft sein, weitere Emulgatoren zu verwenden. So kann beispielsweise die Wasserfestigkeit der erfindungsgemässen Zubereitungen weiter erhöht werden. Geeignete Emulgatoren sind z. B. Alkylmethiconcopolyole und Alkyl-Dimethiconcopolyole, insbesondere Cetyldimethiconcopolyol, Laurylmethiconcopolyol, W/O-Emulgatoren wie Sorbitanstearat, Glycerylstearat, Glycerolstearat, Sorbitanoleat, Lecithin, Glycerylisostearat, Polyglyceryl-3-oleat, Polyglyceryl-3-diisostearat, PEG-7-hydriertes Ricinusoel, Polyglyceryl-4-isostearat, Acrylat/C₁₀₋₃₀-Alkylacrylat-Crosspolymer, Sorbitanisostearat, Poloxamer 101, Polyglyceryl-2-dipolyhydroxystearat, Polyglyceryl-3-diisostearat, Polyglyceryl-4-dipolyhydroxystearat, PEG-30-dipolyhydroxystearat, Diisostearoylpolyglyceryl-3-diisostearat, Glycoldistearat und Polyglyceryl-3-dipolyhydroxystearat.

Die erfindungsgemäßen O/W-Zusammensetzungen können vorteilhaft Verdicker der Wasserphase enthalten. Vorteilhafte Verdicker sind:
- Vernetzte oder nichtvernetzte Acrylsäure- oder Methacrylsäure-Homo- oder Copolymere. Hierzu gehören vernetzte Hompolymere von Methacrylsäure oder Acrylsäure, Copolymerisate aus Acrylsäure und/oder Methacrylsäure und Monomeren, die von anderen Acryl- oder Vinylmonomeren abgeleitet sind, wie C10-30 Alkylacrylate, C10-30-Alkylmethacrylate und Vinylacetat.
- Verdickende Polymere natürlicher Herkunft beispielweise auf Cellulosebasis, Guargummi, Xanthan, Scleroglucan, Gellangummi, Rhamsan und Karayagummi, Alginate, Maltodextrin, Stärke und ihre Derivate, Johannisbrotkernmehl, Hyaluronsäure, Carrageenan.
- Nichtionische, anionische, kationische oder amphotere assoziative Polymere z.B. auf Basis von Polyethylenglycole und ihre Derivate, oder Polyurethane.
- Vernetzte oder nichtvernetzte Homopolymere oder Copolymere auf Basis von Acrylamid oder Methacrylamid, wie Homopolymere von 2-Acrylamido-2-methylpropansulfonsäure, Copolymere von Acrylamid oder Methacrylamid und Methacryloyloxyethyltrimethylammoniumchlorid oder Copolymere von Acrylamid und 2-Acrylamido-2-methylpropansulfonsäure angegeben werden.

Besondere vorteilhafte Verdicker sind verdickende Polymere natürlicher Herkunft, vernetzte Acrylsäure oder Methacrylsäure Homo- oder Copolymere und vernetzte Copolymere von 2-Acrylamido-2-methylpropansulfonsäure.

Ganz besondere vorteilhafte Verdicker sind Xanthangummi, wie die unter den Bezeichnungen Keltrol^{®} und Kelza^{®} von der Firma CP Kelco angebotenen Produkte oder die Produkte der Firma RHODIA mit der Bezeichnung Rhodopol^{®} und Guargummi, wie die unter der Bezeichnung Jaguar^{®} HP105 von der Firma RHODIA erhältlichen Produkte.

Ganz besondere vorteilhafte Verdicker sind auch vernetzte Homopolymere von Methacrylsäure oder Acrylsäure, die von der Firma Lubrizol unter den Bezeichnungen Carbopol^{®} 940, Carbopol^{®} 941, Carbopol^{®} 980, Carbopol^{®} 981, Carbopol^{®} ETD 2001, Carbopol^{®} EDT 2050, Carbopol^{®} 2984, Carbopol^{®} 5984 und Carbopol^{®} Ultrez 10, von der Firma 3V unter den Bezeichnungen Synthalen^{®} K, Synthalen^{®} L und Synthalen^{®} MS und von der Firma PROTEX unter den Bezeichnungen Modarez^{®} V 1250 PX, Modarez^{®} V2000 PX, Viscaron^{®} A1600 PE und Viscaron^{®} A700 PE im Handel erhältlich sind.

Ganz besondere vorteilhafte Verdicker sind vernetzte Copolymer von Acrylsäure oder Methacrylsäure und einem C₁₀₋₃₀-Alkylacrylat oder C₁₀₋₃₀-Alkylmethacrylat und Copolymere von Acrylsäure oder Methacrylsäure und Vinylpyrrolidon. Solche Copolymere sind beispielsweise von der Firma Lubrizol unter den Bezeichnungen Carbopol^{®} 1342, Carbopol^{®} 1382, Pemulen^{®} TR1 oder Pemulen^{®} TR2 und von der Firma ISP unter den Bezeichnungen Ultrathix^{®} P-100 (INCl : Acrylic Acid/VP Crosspolymer) im Handel erhältlich.

Ganz besondere vorteilhafte Verdicker sind vernetzte Copolymere von 2-Acrylamido-2-methylpropansulfonsäure. Solche Copolymere sind beispielsweise von der Firma Clariant unter den Bezeichnungen Aristoflex^{®} AVC (INCl: Ammonium AcryloyldimethyltaurateNP Copolymer) erhältlich

Diese Verdicker sind im Allgemeinen in einer Konzentration von etwa 0 % bis 2 Gew.-% vorzugsweise 0 % bis 1 Gew.-% bezogen auf das Gesamtgewicht der erfindungsgemäßen Zusammensetzung vorhanden.

Weitere erfindungsgemäße Zusammensetzung können Wasser-in-Öl- oder Wasser-in-Silikon-Emulsionen sein. Bevorzugt sind Wasser-in-Öl- (W/O) oder Wasser-in-Silikon-Emulsionen (W/Si), die ein oder mehrere Silikonemulgatoren (W/S) mit einem HLB-Wert ≤ 8 oder ein oder mehrere W/O-Emulgatoren mit einem HLB-Wert < 7 und gegebenenfalls ein oder mehrere O/W-Emulgatoren mit einem HLB-Wert > 10 enthalten.

Die Silikonemulgatoren können vorteilhaft aus der Gruppe enthaltend Alkyldimethiconcopolyole wie z. B. Cetyl PEG/PPG 10/1 Dimethiconcopolyol (ABIL^{®} EM 90 der Fa. Goldschmidt AG) oder Lauryl PEG/PPG- 18/18 Dimethicone (Dow Corning^{®} 5200 der Fa. Dow Corning Ltd.) und Dimethiconecopolyole wie z. B. PEG-10 Dimethicone (KF-6017 der Fa. Shin Etsu), PEG/PPG- 18/18 Dimethicone (Dow Corning 5225C der Fa. Dow Corning Ltd.) oder PEG/PPG-19/19 Dimethicone (Dow Corning BY-11 030 der Fa. Dow Corning Ltd.) gewählt werden.

Die W/O-Emulgatoren mit einem HLB-Wert < 7 können vorteilhaft aus der Gruppe enthaltend Sorbitanstearat, Sorbitanoleat, Glycerylisostearat, Polyglyceryl-3-oleat, Pentaerythrithylisostearat, Methylglucosedioleat, PEG-7-hydriertes Ricinusoel, Polyglyceryl-4-isostearat, Hexyllaurat, Sorbitanisostearat, Polyglyceryl-2-dipolyhydroxystearat, Polyglyceryl-3-Diisostearat, PEG-30-dipolyhydroxystearat, Dii-sostearoylpolyglyceryl-3-diisostearat, Polyglyceryl-3-dipolyhydroxystearat, Polyglyceryl-4-dipolyhydroxystearat, Polyglyceryl-3-dioleat und Wollwachsalkohol (Eucerit) gewählt werden.

Die O/W-Emulgatoren mit einem HLB-Wert > 10 können vorteilhaft aus der Gruppe enthaltend Lecithin, Trilaureth-4-Phosphat, Polysorbate-20, Polysorbate-60, PEG-22-Dodecylglycol Copolymer, Sucrosestearat und Sucroselaurat gewählt werden.
Zur Stabilisierung der erfindungsgemäßen W/O Emulsion gegen Sedimentierung oder Flockung der Wassertröpfchen kann vorteilhaft ein Ölverdicker eingesetzt werden.

Besonders vorteilhaft Ölverdicker sind organomodifizierte Tone wie organomodifizierte Bentonite (Bentone^{®} 34 der Firma Rheox) organomodifizierte Hectorite (Bentone^{®} 27 und Bentone^{®} 38 der Firma Rheox) oder organomodifizierte Montmorillonit, hydrophobe pyrogene Kiesselsäure, wobei die Silanolgruppen mit Trimethylsiloxygruppen substituiert sind (AEROSIL^{®} R812 der Firma Degussa) oder mit Dimethylsiloxygruppen oder Polydimethylsiloxan (AEROSIL^{®} R972, AEROSIL^{®} R974 von Degussa, CAB-O-SIL^{®} TS-610, "CAB-O-SIL^{®} TS-720 von Cabot), Magnesium- oder Aluminiumstearat, oder Styrol Copolymere wie zum Beispiel Styrol-Butadien-Styrol, Styrol-Isopropen-Styrol, Styrol-Ethylen/Buten-Styrol oder Styrol-Ethylen/Propen-Styrol.
Das Verdickungsmittel für die Fettphase kann in einer Menge von 0,1 bis 5 Gew.-%, bezogen auf das Gesamtgewicht der Emulsion, und besser 0,4 bis 3 Gew.-% enthalten sein.

Die wässrige Phase kann ferner Stabilisierungsmittel enthalten. Das Stabilisierungsmittel kann beispielweise Natriumchlorid, Magnesiumchlorid oder Magnesiumsulfat und deren Gemischen sein.

Öle können in W/O-, W/Si- und O/W-Emulsionen eingesetzt werden.

Wenn vorhanden, enthält die Fettphase der erfindungsgemäßen Zusammensetzung mindestens ein nicht flüchtiges Öl. Die Fettphase der Zusammensetzung kann ferner auch flüchtige Öle und Wachse enthalten. Die O/W- Zusammensetzung enthält vorteilhaft 0 bis 45 Gew.% Öle, bezogen auf das Gesamtgewicht der Zusammensetzung, und besonders vorteilhaft 0 bis 20 Gew:% Öle. Die W/O oder W/Si-Zusammensetzung enthält vorteilhaft mindestens 20 Gew.% Öle, bezogen auf das Gesamtgewicht der Zusammensetzung.

Das nicht flüchtige Öl wird vorteilhaft gewählt aus der Gruppe von mineralischer, tierischer, pflanzlicher oder synthetischer Herkunft, polaren oder unpolaren Ölen und deren Gemischen.

Polare Öle können unter der Lecithine und den Fettsäuretriglyceride, namentlich der Triglycerinester gesättigter und/oder ungesättigter, verzweigter und/oder unverzweigter Alkancarbonsäuren einer Kettenlänge von 8 bis 24, insbesondere 12 bis 18 C-Atomen ausgewählt werden. Beipielweise können die Fettsäuretriglyceride gewählt werden aus der Gruppe von Cocoglycerid, Olivenöl, Sonnenblumenöl, Sojaöl, Erdnussöl, Rapsöl, Mandelöl, Palmöl, Kokosöl, Rizinusöl, Weizenkeimöl, Traubenkernöl, Distelöl, Nachtkerzenöl, Macadamianussöl, Aprikosenkernöl, Avocadoöl und dergleichen mehr.

Weitere vorteilhafte polare Öle können gewählt werden aus der Gruppe der Ester aus gesättigten und/oder ungesättigten, verzweigten und/oder unverzweigten Alkancarbonsäuren einer Kettenlänge von 3 bis 30 C-Atomen und gesättigten und/oder ungesättigten, verzweigten und/oder unverzweigten Alkoholen einer Kettenlänge von 3 bis 30 C-Atomen sowie aus der Gruppe der Ester aus aromatischen Carbonsäuren und gesättigten und/oder ungesättigten, verzweigten und/oder unverzweigten Alkoholen einer Kettenlänge von 3 bis 30 C-Atomen. Beispielweise können die Esteröle vorzugsweise gewählt werden aus der Gruppe von Phenethylbenzoat, Octylpalmitat, Octylcocoat, Octylisostearat, Octyldodeceylmyristat, Octyldodekanol, Cetearylisononanoat, Isopropylmyristat, Isopropylpalmitat, Isopropylstearat, Isopropyloleat, n-Butylstearat, n-Hexyllaurat, n-Decyloleat, Diisopropyladipat, Isooctylstearat, Isononylstearat, Isononylisononanoat, 2-Ethylhexylpalmitat, 2-Ethylhexyllaurat, 2-hexyldecyllaurat, 2-Hexyldecylstearat, 2-Octyldodecylpalmitat, 2-Octyldodecylmyristat, 2-Octyldodecyllactat, 2-Diethylhexylsiccint, Diiostearylmalat, Glyceryltrriisostearat, Diglyceryltriisostearat, Stearylheptanoat, Oleyloleat, Oleylerucat, Erucyloleat, Erucylerucat, Tridecylstearat, Tridecyltrimellitat, sowie synthetische, halbsynthetische und natürliche Gemische solcher Ester, wie z.B. Jojobaöl.

Vorteilhaft können die polaren Öle gewählt werden aus der Gruppe der Dialkylether und Dialkylcarbonate, vorteilhaft sind z.B. Dicaprylylether (Cetiol^{®} OE der Firma Cognis) und/oder Dicaprylylcarbonat (beispielsweise Cetiol^{®} CC der Firma Cognis).

Es ist ferner bevorzugt, die polaren Öle aus der Gruppe Isoeikosan, Neopentylglykoldiheptanoat, Propylenglykoldicaprylat/dicaprat, Caprylic/Capric/Diglycerylsuccinat, Butylenglykol Dicaprylat/Dicaprat, C₁₂₋₁₃-Alkyllactat, Di-C₁₂₋₁₃-Alkyltartrat, C12-15 Alkylbenzoat, Myristylmyristat, Isodecylneopentanoat, Triisostearin, Dipentaerythrityl Hexacaprylat/Hexacaprat, Propylenglykolmonoisostearat, Tricaprylin, Dimethylisosorbid, Butyloctylsalicylat (beispielsweise das unter der Handelsbezeichnung Hallbrite^{®} BHB bei der Fa. CP Hall erhältliche), Hexadecylbenzoat und Butyloctylbenzoat und Gemische davon (Hallstar^{®} AB) und/oder Diethylhexylnaphthalat (Hallbrite^{®} TQ oder Corapan^{®} TQ von Symrise) ausgewählt werden.

Das nicht flüchtige Öl kann ebenfalls vorteilhaft auch ein unpolares Öl sein, welche gewählt wird aus der Gruppe der verzweigten und unverzweigten Kohlenwasserstoffe, insbesondere Mineralöl, Vaselineöl, Paraffinöl, Squalan und Squalen, Polyolefine beispielweise Polydecene, hydrogenierte Polyisobutene, C13-16 Isoparaffin und Isohexadecan.

Das unpolare nicht flüchtige Öl kann unter den nicht flüchtigen Silikonölen ausgewählt werden.

Von den nicht flüchtigen Silikonölen können die Polydimethylsiloxane (PDMS), die gegebenenfalls phenyliert sind, wie die Phenyltrimethicon, oder gegebenenfalls substituiert sind mit aliphatischen und/oder aromatischen Gruppen oder mit funktionellen Gruppen, beispielsweise Hydroxygruppen, Thiolgruppen und/oder Aminogruppen; mit Fettsäuren, Fettalkoholen oder Polyoxyalkylenen modifizierte Polysiloxane und deren Gemische angegeben werden.

Die erfindungsgemäße Zusammensetzung kann ferner ein Wachs enthalten.

Im Sinne der vorliegenden Schrift ist ein Wachs als lipophile Fettsubstanz definiert, die bei Raumtemperatur (25°C) fest ist und bei einer Schmelztemperatur zwischen 30°C und 200°C eine reversible Zustandsänderung fest/flüssig zeigt. Oberhalb des Schmelzpunktes wird das Wachs niedrigviskos und mischbar mit Ölen.

Der Wachs wird vorteilhaft gewählt aus der Gruppen von natürlichen Wachsen wie beispielweise Baumwollwachs, Carnaubawachs, Candelillawachs, Espartoawachs, Japanwachs, Montanwachs, Zuckerrohrwachs, Bienenwachs, Wollwachs, Schellack, Mikrowachse, Ceresin, Ozokerit, Ouricuriwachs, Korkfaserwachs, Lignitwachse, Berrenwachs, Sheabutter oder synthetische Wachse wie Paraffinwachse, Polyethylenwachse, durch Fischer-Tropsch-Synthese hergestellte Wachse, hydrierte Öle, Fettsäureester und Glyceride, die bei 25 °C fest sind, Silikonwachse und Derivaten (lkylderivate, Alkoxyderivate und/oder Ester von Polymethylsiloxan) und deren Gemischen. Die Wachse können in Form von stabilen Dispersionen von kolloidalen Wachspartikeln vorliegen, die nach bekannten Verfahren hergestellt werden können, beispielsweise gemäß "Microemulsions Theory and Practice", L.M. Prince Ed., Academic Press (1977), Seiten 21-32.

Die Wachse können in Mengen von 0 bis 10 Gew.-%, bezogen auf das Gesamtgewicht der Zusammensetzung und vorzugsweise 0 bis 5 Gew.-% enthalten sein.

Die erfindungsgemäße Zusammensetzung kann ferner ein flüchtiges Öl enthalten, das aus der Gruppe von flüchtigen Kohlenwasserstoffölen, silikonierten Ölen oder fluorierten Ölen ausgewählt wird.

Das flüchtige Öl kann in einer Menge von 0 bis 25 Gew.-%, bezogen auf das Gesamtgewicht der Emulsion, vorzugsweise 0 bis 20 Gew.-% und noch bevorzugter 0 bis 15 Gew.-% enthalten sein.

Im Sinne der vorliegenden Schrift ist ein flüchtiges Öl ein Öl, das im Kontakt mit der Haut bei Raumtemperatur und Atmosphärendruck in weniger als einer Stunde verdämpft. Das flüchtige Öl ist bei Raumtemperatur flüssig und bei Raumtemperatur und Atmosphärendruck einen Dampfdruck von 0,13 bis 40 000 Pa (10⁻³ bis 300 mg Hg), vorzugsweise 1,3 bis 13 000 Pa (0,01 bis 100 mm Hg) und besonders bevorzugt 1,3 bis 13 00 Pa (0,01 bis 10 mm Hg) und einen Siedepunkt von 150 bis 260 °C und vorzugsweise 170 bis 250 °C besitzt.

Unter einem Kohlenwasserstofföl wird ein Öl verstanden, das im Wesentlichen aus Kohlenstoffatomen und Wasserstoffatomen und gegebenenfalls Sauerstoffatomen oder Stickstoffatomen gebildet wird und keine Siliziumatome oder Fluoratome enthält, wobei es auch aus Kohlenstoffatomen und Wasserstoffatomen bestehen kann; es kann aber auch Estergruppen, Ethergruppen, Aminogruppen oder Amidgruppen enthalten.

Unter einem Silikon-Öl wird ein Öl verstanden, das mindestens ein Siliziumatom und insbesondere Si-O-Gruppen enthält, wie insbesondere Polydiorganosiloxane.
Unter einem fluorierten Öl ist ein Öl zu verstehen, das mindestens ein Fluoratom enthält.

Das erfindungsgemäße flüchtige Kohlenwasserstofföl kann unter den Kohlenwasserstoffölen mit einem Flammpunkt von 40 bis 102 °C, vorzugsweise 40 bis 55 °C und noch bevorzugter 40 bis 50 °C ausgewählt werden.

Beispielweise sind die flüchtigen Kohlenwasserstoffölen solche mit 8 bis 16 Kohlenstoffatomen und deren Gemische, insbesondere verzweigte C₈₋₁₆-Alkane, wie die I-soalkane (die auch als Isoparaffine bezeichnet werden) mit 8 bis 16 Kohlenstoffatomen, Isododecan, Isodecan, Isohexadecan und beispielsweise die Öle, die unter den Handelsnamen Isopars^{®} oder Permetyls^{®} angeboten werden; und die verzweigten C₈₋₁₆-Ester, wie Isohexylneopentanoat und deren Gemische.

Besonders vorteilhaft sind die flüchtigen Kohlenwasserstofföle wie Isododecan, Isodecan und Isohexadecan

Das erfindungsgemäße flüchtige silikonierte Öl kann unter den silikonierten Ölen mit einem Flammpunkt von 40 bis 102 °C, vorzugsweise einem Flammpunkt über 55 °C und höchstens 95 °C und besonders bevorzugt im Bereich von 65 bis 95 °C ausgewählt werden.

Beispielweise sind die flüchtigen silikonierten Öle geradkettigen oder cyclischen Silikonöle mit 2 bis 7 Siliziumatomen genannt werden, wobei diese Silikone gegebenenfalls Alkyl- oder Alkoxygruppen mit 1 bis 10 Kohlenstoffatomen enthalten.

Besonders vorteilhaft sind die flüchtigen silikonierten Öle wie Octamethylcyclotetrasiloxan, Decamethylcyclopentasiloxan, Dodecamethylcyclohexasiloxan, Heptamethylhexyltrisiloxan, Heptamethyloctyltrisiloxan, Hexamethyldisiloxan, Octamethyltrisiloxan, Decamethyltetrasiloxan, Dodecamethylpentasiloxan und deren Gemische. Das flüchtige fluorierte Öl besitzt im Allgemeinen keinen Flammpunkt.

Beispielweise sind die flüchtigen fluorierte Öle Nonafluorethoxybutan, Nonafluormethoxybutan, Decafluorpentan, Tetradecafluorhexan, Dodecafluorpentan und deren Gemische.

Das bevorzugte kosmetische akzeptable Medium der erfindungsgemäßen Zusammensetzung enthält Wasser und gegebenenfalls ein kosmetisch verträgliches in Wasser mischbares geeignetes organisches Lösungsmittel.

Das verwendete Wasser in der erfindungsgemäßen Zusammensetzung kann ein Blütenwasser, reines demineralisiertes Wasser, Mineralwasser, Thermalwasser und/oder Meerwasser sein.

Im Fall einer O/W Zusammensetzung als erfindungsgemäße Zusammensetzung kann der Wasseranteil im Bereich von 40 bis 95 Gew.-%, bevorzugt im Bereich von 50 bis 90 Gew.-%, ganz bevorzugt im Bereich von 60 bis 80 Gew.-% bezogen auf das Gesamtgewicht der Zusammensetzung, liegen. Im Fall einer W/O Zusammensetzung liegt der Wasseranteil im Bereich von 0 bis 60 Gew.-%, bevorzugt im Bereich von 10 bis 50 Gew.-%, ganz bevorzugt im Bereich von 30 bis 50 Gew.-% bezogen auf das Gesamtgewicht der Zusammensetzung.

Die bevorzugten Lösungsmittel sind beispielsweise die aliphatischen Alkohole mit C1-4 Kohlenstoffatomen wie Ethanol und Isopropanol; Polyol und deren Derivate wie Propylenglykol, Dipropylenglykol, Butylen-1,3-glykol, Polypropylenglykol, Glykolether wie Alkyl (C1-4)ether von Mono-, Di- oder Tripropylenglykol oder Mono-; Di- oder Triethylenglykol, und deren Gemischen ausgewählt.

Der Mengenanteil des Lösungsmittels oder der Lösungsmittel in der erfindungsgemäßen Zusammensetzung kann beispielsweise im Bereich von 0 bis 25 Gew.-% und bevorzugt 0 bis 15 Gew.-%, bezogen auf das Gesamtgewicht der Zusammensetzung, liegen.

Weitere erfindungsgemäße Zusammensetzungen können ein "loose Powder" oder ein Kompaktpulver sein.

Die erfindungsgemäße dekorative kosmetische Zusammensetzung kann bevorzugt auch einen sogenannten Foundationeffekt bereitstellen, mittels dessen Unebenheiten der Haut, wie Falten etc. geglättet werden.

Die erfindungsgemäßen dekorativen kosmetischen Zusammensetzungen können weitere Zusatzstoffe enthalten, die in der Kosmetik üblich sind, wie zum Beispiel: Antioxidantien, Lichtschutzmittel und/oder andere Hilfs- und Zusatzmittel wie beispielsweise Emulgatoren, grenzflächenaktive Stoffe, Entschäumer, Verdicker, Tenside, Wirkstoffe, Feuchthaltemittel, sensorische Additiv, UV-Filter, Filmbildner, Lösemittel, Koaleszenzmittel, Aromastoffe, Geruchsabsorber, Parfüms, Gelbildner und/oder andere Polymerdispersionen wie beispielsweise Dispersionen auf Basis von Polyacrylaten enthalten, Füllstoffe, Weichmacher, Pigmente, Verlaufsmittel und/oder Thixotropiemittel, Geschmeidigkeitsmittel, Weichmacher, Konservierungsmittel. Die Mengen der verschiedenen Zusatzstoffe sind dem Fachmann für den einzusetzenden Bereich bekannt und liegen beispielweise im Bereich von 0 bis 25 Gew.-% bezogen auf das Gesamtgewicht der Zusammensetzung.

Die erfindungsgemäße dekorative kosmetische Zusammensetzung kann auch sensorische Additiv enthalten. Unter sensorischen Additiven sind farblose oder weiße, mineralische oder synthetische, lamellare, sphärische oder längliche inerte Partikel oder ein nicht-partikuläres sensorisches Additiv zu verstehen, welche z.B. die sensorischen Eigenschaften der Formulierungen weiter verbessern und beispielsweise ein samtiges oder seidiges Hautgefühl hinterlassen.

Die sensorischen Additive können in der erfindungsgemäßen Zusammensetzung in beispielsweise in einer Menge von 0 bis 10 Gew.-%, bezogen auf das Gesamtgewicht der Zusammensetzung, und vorzugsweise von 0 bis 7 % enthalten sein.

Vorteilhafte partikuläre sensorische Additive im Sinne der vorliegenden Erfindung sind Talkum, Glimmer (Mica), Siliciumdioxid, Kaolin, Stärke und deren Derivaten (beispielweise Tapiocastärke, Distärkephosphat, Aluminium- bzw. Natrium-Stärke Octenylsuccinat und dergleichen), pyrogene Kieselsäure, Pigmente, die weder hauptsächlich UV-Filter-noch färbende Wirkung haben (wie z.B. Bornitrid etc.), Bornitrid, Calciumcarbonat, Dicalciumphosphat, Magnesiumcarbonat, Magnesiumhydrogencarbonat, Hydroxyapatite, mikrokristalline Cellulose, Pulver von synthetischen Polymeren, wie Polyamide (beispielsweise die unter der Handelsbezeichnung "Nylon®" erhältlichen Polymeren), Polyethylen, Poly-β-alanin, Polytetrafluorethylen ("Teflon®"), Polyacrylat, Polyurethan, Lauroyl-Iysine, Silikonharz (beispielsweise die unter der Handelsbezeichnung "Tospearl^{®}" der Firma Kobo Products Inc. erhältlichen Polymeren), Hohlpartikel von Polyvinyliden/Acrylonitrile (Expancel^{®} der Firma Akzo Nobel) oder Hohlpartikel von Siliciumoxid (Silica Beads^{®} der Firma MAPRECOS).

Vorteilhafte nicht-partikuläre sensorische Additive können aus der Gruppe der Dimethiconole (z. B. Dow Corning 1503 Fluid der Fa. Dow Corning Ltd.), der Siliconcopolymere (z. B. Divinyldimethicone/Dimethicone Copolymer, Dow Corning HMW 2220 der Fa. Dow Corning Ltd.) oder der Siliconelastomere (z. B. Dimethicone Crosspolymer, Dow Corning 9040 Silicone Elastomer Blend der Fa. Dow Corning Ltd.) ausgewählt werden.

Die erfindungsgemäße Zusammensetzung kann gegebenenfalls auch Sonnenschutzfilter enthalten, wobei die Gesamtmenge der Sonnenschutzfilter 0 Gew.-% bis 30 Gew.-%, vorteilhaft 0 Gew.-% bis 20 Gew.%, besonders vorteilhaft 0 Gew.-% bis 10 Gew.-% beträgt, bezogen auf das Gesamtgewicht der erfindungsgemäßen Zusammensetzung. Die Sonnenschutzfilter (oder UV-Filter) können insbesondere unter den organischen Filtern, den physikalischen Filtern und deren Gemischen ausgewählt werden.

Die erfindungsgemäße Zusammensetzung kann UV-A-, UV-B-Filter oder Breitbandfilter enthalten. Die eingesetzten UV-Filter öllöslich oder wasserlöslich können sein. Die untere beigefügte Liste der genannten UV-Filter ist selbstverständlich nicht limitierend.

Von den UV-B-Filtern sind beispielsweise zu nennen:
- 1) Salicylsäurederivate, besonders Homomenthylsalicylat, Octylsalicylat und Salicylsäure(4-isopropylbenzyl)ester;
- 2) Zimtsäurederivate, insbesondere 2-Ethylhexyl-p-methoxycinnamat, das von der Firma Givaudan unter der Bezeichnung Parsol MCX^{®} erhältlich ist und 4-Methoxyzimtsäureisopentylester;
- (3) flüssige β,β'-Diphenylacrylatderivate, insbesondere 2-Ethylhexyl α,β'-diphenylacrylat oder Octocrylen, das von der Firma BASF unter der Bezeichnung UVINUL N539^{®} erhältlich ist;
- (4) p-Aminobenzoesäurederivate, insbesondere 4-(dimethylamino)-benzoesäure (2-ethylhexyl)ester, 4-(dimethylamino)benzoesäureamylester;
- (5) 3-Benzylidencampher-Derivate, insbesondere 3-(4-Methylbenzyliden)campher der von der Firma Merck unter der Bezeichnung EUSOLEX 6300^{®} im Handel ist, 3-Benzylidencampher, Benzylidencampher sulfonsäure und Polyacrylamidomethyl Benzylidencampher;
- (6) 2-Phenylbenzimidazol-5-sulfonsäure, die unter der Bezeichnung EUSOLEX 232^{®} von Merck erhältlich ist;
- (7) 1,3,5-Triazinderivate, insbesondere: - 2,4,6-Tris[p-(2'-ethylhexyl-1'-oxycarbonyl)anilino]-1,3,5-triazin, das von der Firma BASF unter der Bezeichnung UVINUL T150^{®} angeboten wird, und - Dioctylbutamidotriazon, das von der Firma Sigma 3V unter der Bezeichnung UVASORB HEB^{®} angeboten wird;
- (8) Ester der Benzalmalonsäure, insbesondere 4-Methoxybenzalmalonsäuredi(2-ethylhexyl)ester und 3-(4-(2,2-bis ethoxy-carbonylvinyl)-phenoxy)propenyl)-methoxysiloxan/ dimethylsiloxan-Copolymer, das von der Firma Roche Vitamines unter der Bezeichnung Parsol^{®} SLX erhältlich ist; und
- (9) die Gemische dieser Filter.

Als UV-A-Filter sind beispielsweise zu nennen:
- (1) Dibenzoylmethanderivate, besonders das 4-(t-Butyl)-4'-methoxydibenzoylmethan, das von der Firma Givaudan unter der Bezeichnung PARSOL 1789^{®} angeboten wird und 1-Phenyl-3-(4'-isopropylphenyl)propan-1,3-dion;
- (2) Benzol-1,4-[di(3-methylidencampher-10-sulfonsäure)], gegebenenfalls ganz oder teilweise neutralisiert, unter der Bezeichnung MEXORYL SX® von Chimex im Handel.
- (3) 2-(4'-Diethylamino-2'-hydroxybenzoyl)benzoesäurehexylester (auch Aminobenzophenon);
- (4) Silanderivate oder Polyorganosiloxane mit Benzophenongruppen;
- (5) Anthranilate, besonders Menthylanthranilat, das von der Firma Symrise unter der Bezeichnung NEO HELIOPAN MA^{®} angeboten wird;
- (6) Verbindungen, die pro Molekül mindestens zwei Benzoazolylgruppen oder mindestens eine Benzodiazolylgruppe enthalten, insbesondere 1,4-Bis-benzimidazolylphenylen-3,3',5,5'-tetrasulfonsäure sowie ihre Salze, die von der Firma Symrise im Handel sind;
- (7) Siliciumderivate von Benzimidazolylbenzazolen, die N-substituiert sind, oder von Benzofuranylbenzazolen, insbesondere: - 2-[1-[3-[1,3,3,3-Tetramethyl-1-[(trimethylsilyl)oxy]disiloxanyl]-propyl]-1H-benz imidazol-2-yl]-benzoxazol; - 2-[1-[3-[1,3,3,3-Tetramethyl-I-[(trimethylsilyl)oxy]disiloxanyl]-propyl]-1H-benz imidazol-2-yl]-benzothiazol; - 2-[1-(3-Trimethylsilanylpropyl)-1 H-benzimidazol-2-yl]-benzoxazol; - 6-Methoxy-1,1'-bis(3-trimethylsilanylpropyl)1H,1'H-[2,2']dibenzimidazolylbenzoxa zol; - 2-[1-(3-Trimethylsilanylpropyl)-1H-benzimidazol-2-yl]-benzothiazol; die in der Patentanmeldung EP-A-1 028 120 beschrieben sind;
- (8) Triazinderivate, insbesondere 2,4-bis-[5-1(dimethylpropyl)benzoxazol-2-yl-(4-phenyl)-imino]-6-(2-ethylhexyl)-imino-1,3,5-triazin, das von der Firma 3V unter der Bezeichnung Uvasorb^{®}K2A angeboten wird; und
- (9) deren Gemische.

Als Breitbandfilter sind beispielweise zu nennen :
- 1) Benzophenonderivate, beispielsweise
   - 2,4-Dihydroxybenzophenon (Benzophenon-1);
   - 2,2',4,4'-Tetrahydroxybenzophenon (Benzophenon-2);
   - 2-Hydroxy-4-methoxybenzophenon (Benzophenon-3), von der Firma BASF unter der Bezeichnung UNIVNUL M40^{®} erhältlich;
   - 2-Hydroxy-4-methoxybenzophenon-5-sulfonsäure (Benzophenon-4), sowie ihre Sulfonatform (Benzonphenon-5), von der Firma BASF unter der Bezeichnung UVINUL MS40^{®} im Handel;
   - 2,2'-Dihydroxy-4,4'-dimethoxybenzophenon (Benzophenon-6-);
   - 5-Chlor-2-hydroxybenzophenon (Benzophenon-7-);
   - 2,2'-Dihydroxy-4-methoxybenzophenon (Benzophenon-8);
   - das Dinatriumsalz von 2,2'-Dihydroxy-4,4'-dimethoxybenzophenon-5,5'-disulfonsäure (Benzophenon-9-);
   - 2-Hydroxy-4-methoxy-4'-methylbenzophenon (Benzophenon-10);
   - Benzophenon-11;
   - 2-Hydroxy-4-(octyloxy)-benzophenon (Benzophenon-12).
- (2) Triazinderivate, insbesondere das 2,4-Bis{[4-2-ethylhexyloxy)-2-hydroxy]-phenyl}-6-(4-methoxyphenyl)-1,3,5-triazin, das von der Firma Ciba Geigy unter der Bezeichnung TINOSORB S^{®} angeboten wird, und das 2,2'-Methylen-bis[6-(2H-benzotriazol-2-yl)4-(1,1,3,3-tetramethylbutyl)phenol], das von der Firma Ciba Geigy unter der Bezeichnung TINOSORB M^{®} erhältlich ist; und
- (3) 2-(1 H-Benzotriazol-2-yl)-4-methyl-6-[2-methyl-3-[1,3,3,3-tetramethyl-1-[(trimethylsilyl)oxy]disiloxanyl]propyl]-phenol mit der INCl-Bezeichnung Dromotrizole Trisiloxane.

Es können auch ein Gemisch von mehreren Filtern und ein Gemisch von UV-B-Filtern, UV-A-Filtern und Breitbandfilter sowie Gemische mit physikalischen Filtern verwendet werden.

Von den physikalischen Filtern können die Sulfat des Bariums, Oxide von Titan (Titandioxid, amorph oder kristallin in Form von Rutil und/oder Anatas), von Zink, von Eisen, von Zirconium, von Cer, Silicium, Mangan oder deren Gemische angegeben werden. Die Metalloxide können in Partikelform mit einer Grösse im Mikrometerbereich oder Nanometerbereich (Nanopigmente) vorliegen. Die mittleren Partikelgrössen betragen für die Nanopigmente beispielsweise 5 bis 100 nm.

Die erfindungsgemäße dekorative kosmetische Zusammensetzung kann ein oder mehrere Feuchthaltemittel (Moisturizer) umfassen.

Besonders vorteilhafte Feuchthaltemittel im Sinne der vorliegenden Erfindung sind beispielsweise Glycerin, Polyglycerin, Sorbit, Dimethylisosorbid, Milchsäure und/oder Lactate, insbesondere Natriumlactat, Butylenglykol, Propylenglykol, Biosaccaride Gum-1, Glycine Soja, Hydroxyethylharnstoff, Ethylhexyloxyglycerin, Pyrrolidoncarbonsäure und Harnstoff. Ferner ist es insbesondere von Vorteil, polymere Moisturizer aus der Gruppe der wasserlöslichen und/oder in Wasser quellbaren und/oder mit Hilfe von Wasser gelierbaren Polysaccharide zu verwenden. Insbesondere vorteilhaft sind beispielsweise Hyaluronsäure, Chitosan und/oder ein fucosereiches Polysaccharid, welches unter der Bezeichnung Fucogel™ 1000 von der Gesellschaft SOLA-BIA S.A. erhältlich ist. Besonders vorteilhaft im Sinne der vorliegenden Erfindung können wasserlösliche Antioxidantien eingesetzt werden, wie beispielsweise Vitamine, z.B. Ascorbinsäure und deren Derivate. Ganz besonders vorteilhafte sind Vitamin E und dessen Derivate sowie Vitamin A und dessen Derivate.

Weitere vorteilhafte Wirkstoffe in der erfindungsgemäßen Zusammensetzung sind α-Hydroxysäure wie Glykolsäure, Milchsäure, Äpfelsäure, Weinsäure, Citronensäure und Mandelsäure, β-Hydroxysäure wie Salicylsäure sowie deren acylierten Derivate, die 2-Hydroxyalkansäure und ihre Derivate; natürliche Wirkstoffe und/oder deren Derivate, wie z.B. alpha-Liponsäure, Folsäure, Phytoen, D-Biotin, Coenzym Q10, alpha-Glucosylrutin, Carnitin, Carnosin, natürliche und/oder synthetische Isoflavonoide, Kreatin, Kreatinin, Taurin und/oder [beta]-Alanin sowie 8-Hexadecen-1,16-dicarbonsäure (Dioic acid, CAS-Nummer 20701-68-2; vorläufige INCl-Bezeichnung Octadecendioic acid) und/oder Licochalcon A und die Pflanzenextrakte.

Vorteilhafte Filmbildner sind Trimethylsiloxysilicate, Silikon Acrylate Copolymere (z. B. TIB4-200 der Fa. Dow Corning oder KP-561 der Fa. Shin Etsu), Trimethyl Pentaphenyl Trisiloxane (Dow Corning 555 Cosmetic Fluid der Fa. Dow Corning Ltd.) oder Vinylpyrrolidon-Copolymer (z.B. PVP/Eicosen-copolymer oder PVP/hexadecancopolymer).

Die vorliegende Erfindung wird an Hand von Beispielen erläutert, wobei sie nicht als einschränkend zu verstehen sind. Alle Mengenangaben, Anteile und Prozentanteile sind, soweit nicht anders angegeben, auf das Gewicht und die Gesamtmenge bzw. auf das Gesamtgewicht der Zusammensetzungen bezogen.

### Beispiele:

Sofern nicht abweichend gekennzeichnet, beziehen sich alle Prozentangaben auf das Gewicht.

Sofern nicht abweichend vermerkt, beziehen sich alle analytischen Messungen auf Messungen bei Temperaturen von 23°C.

Die Feststoff- bzw. Festkörpergehalte werden ermittelt durch Erhitzen einer ausgewogenen Probe auf 125°C bis zur Gewichtskonstanz. Bei Gewichtskonstanz wird durch erneutes Auswiegen der Probe der Festkörpergehalt berechnet.
NCO-Gehalte wurden, wenn nicht ausdrücklich anders erwähnt, volumetrisch gemäß DIN-EN ISO 11909 bestimmt.

Die Kontrolle auf freie NCO-Gruppen wurde mittels IR-Spektroskopie (Bande bei 2260 cm⁻¹) durchgeführt.

Die angegebenen Viskositäten wurden mittels Rotationsviskosimetrie nach DIN 53019 bei 23°C mit einem Rotationsviskosimeter der Firma Anton Paar Germany GmbH, Ostfildern, DE bestimmt.

Die Bestimmung der mittleren Teilchengrößen (angegeben ist das Zahlenmittel) der Polyurethan-Dispersionen erfolgte nach Verdünnen mit entionisiertem Wasser mittels Laserkorrelations-Spektroskopie (Gerät: Malvern Zetasizer 1000, Malver Inst. Limited).

### Verwendete Substanzen und Abkürzungen:

- Diaminosulfonat:: NH₂-CH₂CH₂-NH-CH₂CH₂-SO₃Na (45 %ig in Wasser)
- Desmophen® 2020/C2200:: Polycarbonatpolyol, OH-Zahl 56 mg KOH/g, zahlenmittleres Molekulargewicht 2000 g/mol (Bayer MaterialScience AG, Leverkusen, DE)
- PolyTHF®2000:: Polytetramethylenglykolpolyol, OH-Zahl 56 mg KOH/g, zahlenmittleres Molekulargewicht 2000 g/mol (BASF AG, Ludwigshafen, DE)
- PolyTHF® 1000:: Polytetramethylenglykolpolyol, OH-Zahl 112 mg KOH/g, zahlenmittleres zahlenmittleres Molekulargewicht 1000 g/mol (BASF AG, Ludwigshafen, DE)
- Polyether LB 25:: monofunktioneller Polyether auf Ethylenoxid-/Propylen-oxidbasis zahlenmittleres Molekulargewicht 2250 g/mol, OH-Zahl 25 mg KOH/g (Bayer MaterialScience AG, Leverkusen, DE)

### Beispiel 1: Polyurethan-Dispersion 1

987,0 g PolyTHF^{®} 2000 (Komponente A2)), 375,4 g PolyTHF^{®} 1000 (Komponente A2)), 761,3 g Desmophen^{®} C2200 (Komponente A2)) und 44,3 g Polyether LB 25 (Komponente A4)) wurden in einer Standard-Rührapparatur auf 70°C aufgeheizt. Anschließend wurde ein Gemisch aus 237,0 g Hexamethylendiisocyanat (Komponente A1)) und 313,2 g Isophorondiisocyanat (Komponente A1)) zugegeben und bei 120°C gerührt, bis der theoretische NCO-Wert erreicht war. Das fertige Prepolymer wurde mit 4830 g Aceton gelöst und dabei auf 50°C abgekühlt und anschließend wurde eine Lösung aus 25,1 g Ethylendiamin (Komponente B1)), 116,5 g Isophorondiamin (Komponente B1)), 61,7 g Diaminosulfonat (Komponente B2)) und 1030 g Wasser zudosiert. Die Nachrührzeit betrug 10 min. Dann wurde durch Zugabe von 1250 g Wasser dispergiert. Es folgte die Entfernung des Lösemittels durch Destillation im Vakuum.

Die erhaltene weiße Dispersion hatte nachfolgende Eigenschaften:

| | |
|---|---|
| Feststoffgehalt: | 61% |
| Partikelgröße (LKS): | 312 nm |
| Viskosität (Viskosimeter, 23°C): | 241 mPas |
| pH (23°C): | 6,02 |
| pH (23°C): | 7,15 |

### Beispiel 2: Polyurethan-Dispersion 2

450 g PolyTHF^{®} 1000 (Komponente A2)) und 2100 g PolyTHF^{®} 2000 (Komponente A2)) wurden auf 70°C aufgeheizt. Anschließend wurde ein Gemisch aus 225,8 g Hexamethylendiisocyanat (Komponente A1)) und 298,4 g Isophorondiisocyanat (Komponente A1)) zugegeben und solange bei 100-115°C gerührt bis der theoretische NCO-Wert unterschritten war. Das fertige Prepolymer wurde mit 5460 g Aceton bei 50°C gelöst und anschließend eine Lösung aus 29,5 g Ethylendiamin (Komponente B1)), 143,2 g Diaminosulfonat (Komponente B2)) und 610 g Wasser zudosiert. Die Nachrührzeit betrug 15 min. Danach wurde durch Zugabe von 1880 g Wasser dispergiert. Es folgte die Entfernung des Lösemittels durch Destillation im Vakuum und es wurde eine lagerstabile Dispersion erhalten.

| | |
|---|---|
| Feststoffgehalt: | 56 % |
| Partikelgröße (LKS): | 276 nm |
| Viskosität: | 1030 mPas |

### Beispiel 3: Polyurethan-Dispersion 3

1649,0 g eines Polyesters aus Adipinsäure, Hexandiol und Neopentylglykol mit einem mittleren Molekulargewicht von 1700 g/mol (Komponente A2)) wurden auf 65°C aufgeheizt. Anschließend wurden 291,7 g Hexamethylendiisocyanat (Komponente A1)) zugegeben und solange bei 100-115°C gerührt bis der theoretische NCO-Wert unterschritten war. Das fertige Prepolymer wurde mit 3450 g Aceton bei 50°C gelöst und anschließend eine Lösung aus 16,8 g Ethylendiamin (Komponente B1)), 109,7 g Diaminosulfonat (Komponente B2)) und 425 g Wasser zudosiert. Die Nachrührzeit betrug 15 min. Danach wurde durch Zugabe von 1880 g Wasser dispergiert. Es folgte die Entfernung des Lösemittels durch Destillation im Vakuum und es wurde eine lagerstabile Dispersion erhalten.

| | |
|---|---|
| Feststoffgehalt: | 42 % |
| Partikelgröße (LKS): | 168 nm |
| Viskosität: | 425 mPas |
| pH-Wert: | 7,07 |

### Beispiel 4: Polyurethan-Dispersion 4

340 g eines Polyesters aus Adipinsäure, Hexandiol und Neopentylglykol mit einem mittleren Molekulargewicht von 1700 g/mol (Komponente A2)) wurden auf 65°C aufgeheizt. Anschließend wurden 60,1 g Hexamethylendiisocyanat (Komponente A1)) zugegeben und solange bei 105°C gerührt bis der theoretische NCO-Wert unterschritten war. Das fertige Prepolymer wurde mit 711 g Aceton bei 50°C gelöst und anschließend eine Lösung aus 2,1 g Ethylendiamin (Komponente B1)), 32,4 g Diaminosulfonat (Komponente B2)) und 104,3 g Wasser zudosiert. Die Nachrührzeit betrug 15 min. Danach wurde durch Zugabe von 1880 g Wasser dispergiert. Es folgte die Entfernung des Lösemittels durch Destillation im Vakuum und es wurde eine lagerstabile Dispersion erhalten.

| | |
|---|---|
| Feststoffgehalt: | 40 % |
| Partikelgröße (LKS): | 198 nm |
| Viskosität: | 700 m Pas |
| pH-Wert: | 6,31 |

### Beispiel 5: Polyurethan-Dispersion 5

450 g PolyTHF^{®} 1000 (Komponente A2)) und 2100 g PolyTHF^{®} 2000 (Komponente A2)) wurden auf 70°C aufgeheizt. Anschließend wurde ein Gemisch aus 225,8 g Hexamethylendiisocyanat (Komponente A1)) und 298,4 g Isophorondiisocyanat (Komponente A1)) zugegeben und solange bei 100-115°C gerührt bis der theoretische NCO-Wert unterschritten war. Das fertige Prepolymer wurde mit 5460 g Aceton bei 50°C gelöst und anschließend eine Lösung aus 351 g Diaminosulfonat (Komponente B2)) und 610 g Wasser zudosiert. Die Nachrührzeit betrug 15 min. Danach wurde durch Zugabe von 1880 g Wasser dispergiert. Es folgte die Entfernung des Lösemittels durch Destillation im Vakuum und es wurde eine lagerstabile Dispersion erhalten.

| | |
|---|---|
| Feststoffgehalt: | 40 % |
| Viskosität: | 1370 mPas |

### Beispiele für kosmetische Formulierungen:

### a) Mascara

| Rohstoffe | Gew.% (bezogen auf die kosmetische Zusammensetzung) |
|---|---|
| Isododecane | 20,00 |
| D5 Cyclomethicone | 5,00 |
| Carnauba Wax | 6,00 |
| Trimethyl Siloxysilicate | 0,75 |
| Dimethicone 200/200 | 10,00 |
| Erfindungsgemäßes Polyurethane (bezogen auf Feststoff in der Polyurethandispersion) | 3,0 |
| Ceresin Wax SP252 | 3,00 |
| Paraffin Wax 130/135 | 3,50 |
| Polyethylene | 2,50 |
| Nylon-12 | 2,00 |
| Silica | 2,00 |
| Stearic Acid | 1,00 |
| Bentone Gel in Isododecane | 15,00 |
| Phenoxyethanol | 1,00 |
| Black Iron Oxide LC989 EM | 10,00 |
| White Beeswax | 1,75 |
| Deionized Water | Ad. 100 |
| Magnesium Aluminum Silicate | 0,50 |
| Triethanolamine 99 % | 0,90 |
| Net-DTB (10% in Butylene Glycol) | 1,00 |

### b) Foundation

| Rohstoffe | Gew.% (bezogen auf die kosmetische Zusammensetzung) |
|---|---|
| Deionized Water | ad. 100 |
| Cellulose Gum | 0,30 |
| Magnesium Aluminum Silicate | 0,35 |
| Lecithin | 0,40 |
| Triethanolamine 99% | 1,25 |
| Butylene Glycol | 6,00 |
| Titanium Dioxide (Water Dispersible) | 8,00 |
| Red Iron Oxide | 0,40 |
| Yellow lron Oxide | 0,80 |
| Black Iron Oxide | 0,10 |
| Colloidal Kaolin | 2,00 |
| Methyl Paraben | 0,20 |
| Isoeicosane | 10,00 |
| Isostearic Acid | 1,00 |
| Stearic Acid | 2,50 |
| Glyceryl Stearate | 1,50 |
| Tridecyl Trimellitate | 1,00 |
| Glyceryl Stearate SE | 1,00 |
| Propyl Paraben | 0,20 |
| Erfindungsgemäßes Polyurethane (bezogen auf Feststoff in der Polyurethandispersion) | 5,0 |
| Wirkstoffe | q.s. |
| Farbstoffe | q.s. |
| Parfüm | q.s. |
| Konservierungsmittel | q.s. |
| Aqua | Ad.100 |

### c) Eyeliner

| Rohstoffe | Gew.% (bezogen auf die kosmetische Zusammensetzung) |
|---|---|
| Oleyl Alcohol | 0,5 |
| Propylene Glycol | 7,5 |
| Xanthan Gum | 0,1 |
| Silica | 0,1 |
| Erfindungsgemäßes Polyurethane (bezogen auf Feststoff in der Polyurethandispersion) | 2,0 |
| Wirkstoffe | q.s. |
| Farbstoffe | q.s. |
| Parfüm | q.s. |
| Konservierungsmittel | q.s. |
| Aqua | Ad.100 |

### d) Bräunungsmittel

| Rohstoffe | Gew.% (bezogen auf die kosmetische Zusammensetzung) |
|---|---|
| Dihydroxyaceton | 3,0 |
| Glycerin | 8,0 |
| Cetyl Alcohol | 0,5 |
| Silica | 3,0 |
| Methylglucose Sesquistearate | 2,0 |
| PEG-100 stearate | 1,0 |
| Cyclomethicone | 4,0 |
| Erfindungsgemäßes Polyurethane (bezogen auf Feststoff in der Polyurethandispersion) | 2,0 |
| Octyldodecanol | 3,0 |
| Dicaprylylcarbonate | 2,0 |
| EDTA | 1,0 |
| Xanthan Gum | 0,3 |
| Natrium Citrat | 0,4 |
| Citronsäure | 0,3 |
| Vitamin E- Acetat | 0,5 |
| Wirkstoffe | q.s. |
| Farbstoffe | q.s. |
| Parfüm | q.s. |
| Konservierungsmittel | q.s. |
| Aqua | Ad.100 |

### e) Getönte Tagescreme

| Rohstoffe | Gew.% (bezogen auf die kosmetische Zusammensetzung) |
|---|---|
| Glyceryl Stearate Citrate | 3,5 |
| Octyldodecanol | 3,0 |
| Cyclomethicone | 3,0 |
| Cetearyl Alcohol | 1,5 |
| Squalane | 2,0 |
| Shea butter | 5,0 |
| Carbomer | 0,5 |
| Glycerin | 10,0 |
| 4-Methylbenzyliden Campher | 5,0 |
| Octyl Methoxycinnamat | 2,5 |
| Octocrylen | 6,0 |
| Butyl Methoxydibenzoylmethane | 2,5 |
| Erfindungsgemäßes Polyurethane (bezogen auf Feststoff in der Polyurethandispersion) | 5,0 |
| EDTA | 1,0 |
| Wirkstoffe | q.s. |
| Farbstoffe | q.s. |
| Parfüm | q.s. |
| Konservierungsmittel | q.s. |
| Aqua | Ad.100 |

### f) Lippenstift

| Rohstoffe | Gew.% (bezogen auf die kosmetische Zusammensetzung) |
|---|---|
| Rizinus oil | 3,0 |
| Caprylic/Capric Triglycerides | 3,0 |
| Octyldodecanol | 5,0 |
| Hydrogenated polyisobutene | 3,0 |
| Jojaba oil | 1,0 |
| Lanolin oil | 1,0 |
| PEG 45 / Dodecyl Glycol copolymer | 2,0 |
| Polyglyceryl-3 Diisostearat | 2,4 |
| Cetyl palmitate | 1,0 |
| C20-40 Alkyl Stearate | 8,0 |
| Carnauba wax | 2,0 |
| Microcristalline wax | 8,0 |
| Glycerin | 10,0 |
| Erfindungsgemäßes Polyurethane (bezogen auf Feststoff in der Polyurethandispersion) | 15,0 |
| Wirkstoffe | q.s. |
| Farbstoffe | q.s. |
| Parfüm | q.s. |
| Konservierungsmittel | q.s. |
| Aqua | Ad.100 |

### Vergleichversuch:

Die erfindungsgemäßen Polyurethane werden gegenüber PVP/Eicosene-Copolymer (Formulierung gemäß nachstehender Tabelle) in einer Mascara-Formulierung verglichen.

| Rohstoffe | Gew.% (bezogen auf die kosmetische Zusammensetzung) | |
|---|---|---|
| | **1** | **2** |
| Isododecane | 20,00 | 20,00 |
| D5 Cyclomethicone | 5,00 | 5,00 |
| Carnauba Wax | 6,00 | 6,00 |
| Trimethyl Siloxysilicate | 0,75 | 0,75 |
| Dimethicone 200/200 | 10,00 | 10,00 |
| PVP/Eicosene Copolymer | 7,50 | |
| Polyurethan aus Beispiel 3 | | 3,0 |
| Ceresin Wax SP252 | 3,00 | 3,00 |
| Paraffin Wax 130/135 | 3,50 | 3,50 |
| Polyethylene | 2,50 | 2,50 |
| Nylon-12 | 2,00 | 2,00 |
| Silica | 2,00 | 2,00 |
| Stearic Acid | 1,00 | 1,00 |
| Bentone Gel in Isododecane | 15,00 | 15,00 |
| Phenoxyethanol | 1,00 | 1,00 |
| Black lron Oxide LC989 EM | 10,00 | 10,00 |
| White Beeswax | 1,75 | 1,75 |
| Deionized Water | Ad. 100 | Ad. 100 |
| Magnesium Aluminum Silicate | 0,50 | 0,50 |
| Triethanolamine 99 % | 0,90 | 0,90 |
| Net-DTB (10% in Butylene Glycol) | 1,00 | 1,00 |

200 µm-Filme aus den oben beschriebenen Formulierungen werden auf eine Glasplatte bei 35 °C gezogen. Die Filme werden 24 h bei 30 °C trocknen lassen. Zur Messung der Wasserbeständigkeit werden die Glassplatten in einem Wasserbad bei RT unter Rühren während 4 Stunden aufgetaucht. Die Wasserbeständigkeit ist proportional zu der gebliebenen Filmfläche.

Ergebnisse des Vergleichs der oben beschriebenen Mascara-Formulierung mit einer erfindungsgemäßen Formulierung gemäß Beispiel 3:

| Rohstoffe | Gew.% (bezogen auf die kosmetische Zusammensetzung) | |
|---|---|---|
| | **1** | **2** |
| Filmbildner | PVP/Eicosene-Copolymer | Polyurethan aus Beispiel 3 |
| Verbliebene Fläche (%) | 20 | 100 |
| Wasserfestigkeit | schlecht | ausgezeichnet |

## Patentansprüche

1. Dekorative kosmetische Zusammensetzung, enthaltend mindestens ein Polyurethan, erhältlich durch Umsetzung eines oder mehrerer wasserunlöslicher, nicht wasserdispergierbarer, isocyanatfunktioneller Polyurethanprepolymere A) mit einer oder mehreren aminofunktionellen Verbindungen B).

2. Dekorative kosmetische Zusammensetzung, enthaltend mindestens ein Polyurethan, erhältlich durch Umsetzung eines oder mehrerer isocyanatfunktioneller Polyurethanprepolymere A), welche im Wesentlichen weder ionische noch ionogene Gruppen aufweisen, mit einer oder mehreren aminofunktionellen Verbindungen B).

3. Dekorative kosmetische Zusammensetzung nach Anspruch 1 oder 2, worin die aminofunktionellen Verbindungen B) aus primären und/oder sekundären Aminen und/oder Diaminen ausgewählt werden.

4. Dekorative kosmetische Zusammensetzung nach einem der Ansprüche 1 bis 3, worin die aminofunktionelle Verbindungen B) mindestens ein Diamin umfassen.

5. Dekorative kosmetische Zusammensetzung nach einem der Ansprüche 1 bis 4, worin die aminofunktionellen Verbindungen B) ausgewählt werden aus aminofunktionellen Verbindungen B2), die ionische und/oder ionogene Gruppen aufweisen, und aminofunktionellen Verbindungen B1), die keine ionische und/oder ionogene Gruppen aufweisen.

6. Dekorative kosmetische Zusammensetzung nach einem der Ansprüche 1 bis 5, worin die aminofunktionellen Verbindungen B) wenigstens eine aminofunktionelle Verbindung B2) umfassen, die ionische und/oder ionogene Gruppen aufweist, bevorzugt 2-(2-Aminoethylamino)ethansulfonsäure und/oder deren Salze.

7. Dekorative kosmetische Zusammensetzung nach einem der Ansprüche 1 bis 6, worin die aminofunktionellen Verbindungen B) wenigstens eine aminofunktionelle Verbindung B1) umfassen, die keine ionische und/oder ionogene Gruppen aufweisen, bevorzugt ein Diamin, dass keine ionischen und/oder ionogenen Gruppen aufweist.

8. Dekorative kosmetische Zusammensetzung nach einem der Ansprüche 1 bis 7, worin die aminofunktionellen Verbindungen B) sowohl aminofunktionelle Verbindungen B2), die ionische und/oder ionogene Gruppen aufweisen, als auch aminofunktionellen Verbindungen B1), die keine ionischen und/oder ionogenen Gruppe aufweisen, umfassen.

9. Dekorative kosmetische Zusammensetzung nach einem der Ansprüche 1 bis 8, worin die Prepolymere A) erhältlich sind durch die Umsetzung von einem oder mehreren Polyolen, ausgewählt aus der Gruppe, die aus Polyether-Polyolen, Polycarbonatpolyolen, Polyether-Polycarbonat-Polyolen und/oder Polyesterpolyolen besteht, und einem oder mehreren Polyisocyanaten.

10. Dekorative kosmetische Zusammensetzung nach einem der Ansprüche 1 bis 9, worin das Polyurethan mindestens eine Sulfonsäure und/oder Sulfonatgruppe, bevorzugt eine Natriumsulfonatgruppe enthält.

11. Dekorative kosmetische Zusammensetzung nach einem der Ansprüche 1 bis 10, **dadurch gekennzeichnet, dass** sie einen oder mehrere Bestandteile enthält, die einen dekorativen Effekt, wie einen farbgebenden oder einen effektgebenden Effekt erzeugen.

12. Verwendung einer Zusammensetzung, enthaltend mindestens ein Polyurethan, erhältlich durch Umsetzung eines oder mehrerer wasserunlöslicher, nicht wasserdispergierbarer, isocyanatfunktioneller Polyurethanprepolymere A) mit einer oder mehreren aminofunktionellen Verbindungen B) als dekorative kosmetische Zusammensetzung.

13. Verwendung von Polyurethanen, erhältlich durch Umsetzung eines oder mehrerer wasserunlöslicher, nicht wasserdispergierbarer, isocyanatfunktioneller Polyurethanprepolymere A) mit einer oder mehreren aminofunktionellen Verbindungen B) zur Herstellung von dekorativen kosmetischen Zusammensetzungen.

14. Kosmetisches Verfahren zum Erzeugen eines dekorativen Effekts auf Haut und/oder Haar, welches das Auftragen einer Zusammensetzung, enthaltend mindestens ein Polyurethan, erhältlich durch Umsetzung eines oder mehrerer wasserunlöslicher, nicht wasserdispergierbarer, isocyanatfunktioneller Polyurethanprepolymere A) mit einer oder mehreren aminofunktionellen Verbindungen B), auf die Haut und/oder Haar umfasst.

15. Kosmetisches Verfahren nach Anspruch 14, worin die Zusammensetzung nach dem Auftrag auf die Haut zumindest teilweise auf dieser verbleibt.
